(19) Europäisches Patentamt
European Patent Office
Office européen des brevets

(11) EP 0 464 638 B1

(12) EUROPÄISCHE PATENTSCHRIFT

(45) Veröffentlichungstag und Bekanntmachung des Hinweises auf die Patenterteilung:
**02.04.1997 Patentblatt 1997/14**

(51) Int Cl.6: **C07H 21/00**, C07H 21/04, A61K 31/70

(21) Anmeldenummer: **91110570.8**

(22) Anmeldetag: **26.06.1991**

# (54) Oligonucleotid-analoge mit terminalen 3'-3'-bzw. 5'-5'-Internucleotidverknüpfungen

Oligonucleotide analogues with terminal 3'-3' or 5'-5'-internucleotide linkages

Analogues d'oligonucléotides avec liaisons terminals 3'-3'-resp. 5'-5'-internucléotidiques

(84) Benannte Vertragsstaaten:
**AT BE CH DE DK ES FR GB GR IT LI LU NL SE**

(30) Priorität: **02.07.1990 DE 4021019**

(43) Veröffentlichungstag der Anmeldung:
**08.01.1992 Patentblatt 1992/02**

(73) Patentinhaber: **HOECHST AKTIENGESELLSCHAFT**
**65926 Frankfurt am Main (DE)**

(72) Erfinder:
- **Rösch, Hannelore**
  **W-7900 Ulm (DE)**
- **Fröhlich, Anja**
  **W-7901 Illerkirchberg (DE)**
- **Ramalho-Ortigao, Jose Flavio**
  **W-7900 Ulm (DE)**
- **Montenarh, Matthias, Prof.Dr.**
  **W-7913 Senden-Ay (DE)**
- **Seliger, Hartmut, Prof.Dr.**
  **W-7915 Elchingen-Thalfingen (DE)**

(56) Entgegenhaltungen:
**EP-A- 0 136 543** **WO-A-89/09221**
**WO-A-90/12022**

- **NUCLEOSIDES & NUCLEOTIDES, 6(1&2), 1987, H. SELIGER et al., Seiten 483-484**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Als antisense-Oligonucleotide bezeichnet man Nucleinsäurefragmente, deren Sequenz komplementär zur codierenden oder "sense"-Sequenz einer Messenger-RNA bzw. zum codogenen Strang der DNA ist. Solche Oligonucleotide finden in wachsendem Maße Anwendung zur Inhibition der Genexpression in vitro oder in Zellkultursystemen. Die Anwendung solcher Substanzen im medizinischtherapeutischen Bereich, z.B. als antivirale Pharmaka, ist Gegenstand umfangreicher Untersuchungen. In der Biologie sind seit längerem schon antisense-RNA-Sequenzen als natürlich auftretende Regulatoren der Genexpression in Prokaryonten (T. Mizuno, M.-Y. Chou and M. Inouye, Proc. Natl. Acad. Sci. USA 81, 1966-1970 (1984)) und auch in Eukaryonten (S.M. Heywood, Nucleic Acids Res. 14, 6771-6772 (1986)) bekannt. Sie bewirken eine Inbibierung der Translation durch Hybridisierung an eine entsprechende Messenger-RNA. In zahlreichen Experimenten konnte inzwischen gezeigt werden, daß solche antisense-RNA-Sequenzen, wenn sie in Bakterien (A. Hirashima, S. Sawaki, Y. Inokuchi and M. Inouye, PNAS USA 83, 7726-7730 (1986)) oder Eukaryontenzellen (J.G. Izant and H. Weintraub, Cell 36, 1007-1015 (1984)) eingeschleust wurden, auch dort die Genexpression behindern können und sowohl antivirale (L.-J. Chang and C.M. Stoltzfus, J. Virology 61, 921-924 (1987)) als auch antionkogen-Wirkung (J.T. Holt, T.V. Gopal, A.D. Moutton and A.W. Nienhuis, PNAS USA 83, 4794-4798 (1986)) zeigen. Für solche Untersuchungen haben synthetische Oligonucleotide gegenüber biologischer antisense-RNA den Vorteil größerer Stabilität und leichterer Zugänglichkeit. Letzteres bezieht sich auf die in neuerer Zeit stark verbesserten Synthesetechniken, die heute kürzere Oligomere (von z.B. 12-30 Basen) relativ leicht zugänglich machen (E. Sonveaux, Bioorganic Chemistry 14, 274-325 (1986); Oligodeoxynucleotides, Antisense Inhibitors of Gene Expression, J.S. Cohen, ed., Macmillan Press, 1989; S. 7ff).

Es hat sich gezeigt, daß schon solche kurzen Oligomeren als Expressionsmodulatoren wirksam sind. Daneben können solche Oligonucleotide auch durch Bindung an den DNA-Doppelstrang wirken unter Ausbildung einer Tripel-Helix. Damit beide, die Antisense Oligonucleotide und die Triplex-Forming Oligonucleotide in biologischen Systemen eingesetzt werden können, müssen allerdings folgende Voraussetzungen erfüllt sein (Oligodeoxynucleotides, Antisense Inhibitors of Gene Expression, J.S. Cohen ed. Macmillian Press, 1989, S. 1ff):

1. sie müssen einerseits gut wasserlöslich sein, andererseits aber die lipophile Zellmembran leicht passieren,

2. sie müssen innerhalb der Zelle hinreichend abbaustabil, d.h. stabil gegen Nucleasen, sein,

3. sie müssen mit intrazellulären Nucleinsäuren bei physiologischen Temperaturen stabile Hybride bilden,

4. die Hybridisierung muß selektiv sein; der Unterschied der Dissoziationstemperatur zu einem Oligonucleotid, das eine Fehlpaarung ergibt, muß hinreichend groß sein, so daß letzteres noch spezifisch abgewaschen werden kann.

1978 konnten Zamecznik und Stephenson, PNAS USA 75, 280-284 und 285-288 (1978)) erstmals zeigen, daß unmodifizierte Oligonucleotide die Vermehrung von Rous Sarcom-Viren hemmen können. Die Oligonucleotide wurden allerdings in sehr hohen Konzentrationen angewandt, zudem wurden die Experimente zumeist in vorher erhitzten Medien durchgeführt, um Nucleasen zu inaktivieren. Die Notwendigkeit dieser Maßnahmen erklärt sich aus dem raschen enzymatischen Abbau, dem Fremdnucleinsäuren im Serum und in Zellen unterworfen sind.

Man hat daher schon früh Untersuchungen durchgeführt mit dem Ziel, Oligonucleotide strukturell so zu modifizieren, daß sie die obengenannten Anforderungen besser erfüllen, insbesondere gegen Nuclease-Abbau besser geschützt sind. Diese Untersuchungen konzentrieren sich vor allem auf eine Modifikation der Phosphodiester-Internucleotidbindung, da diese letzten Endes Angriffspunkt aller Nucleasen ist. Man kann die strukturmodifizierten Internucleotidbindungen grundsätzlich einteilen in

a) Nucleosidverknüpfungen mit Phosphor als Zentralatom.
Diese wiederum können sein:
ionisch/chiral
ionisch/achiral
neutral/chiral

b) neutral/achirale phosphorfreie Nucleosidverknüpfungen.

Zu den strukturmodifizierten, nach wie vor jedoch ionischen Internucleosidverknüpfungen gehören Thiophosphat- und Dithiophosphatverknüpfungen. Thiophosphatmodifizierte Oligonucleotide (Oligodeoxynucleotiden, Antisense Inhibitors of Gene Expression, J.S. Cohen. ed. Macmillan Press, 1989, S 97ff) zeigen zur Zeit die besten Inhibierungseffekte der Genexpression in Zellkulturen, allerdings scheint diese Inhibierung nicht an eine spezifische Sequenz gebunden zu sein, da auch solche thiophosphatmodifizierten Oligonucleotide Inhibierungseffekte zeigen, die nur aus einem Nucleinsäurebaustein bestehen, z.B. Thiophosphatanaloge des Oligocytidylats. Begrenzend für den Einsatz thiophosphatmodifizierter Oligonucleotide ist weiterhin deren Chiralität.

Da bei der Darstellung eines Oligonucleotids von n Basen $2^{n-1}$ Diastereomere gebildet werden, hat jedoch nur der geringste Anteil eines Thiophosphatbindungen enthaltenden Präparats gute Hybridisierungseigenschaften.

Bei Oligonucleotiden mit Dithiophosphat-Internucleosidbindungen (A. Grandas, William S. Marshall, John Nielsen und Martin H. Caruthers, Tetrahedron Lett. 20, 543-546 (1989), G.M. Porritt, C.B. Reese, Tetrahedron Lett. 30, 4713-4716 (1989)), wird das Chiralitätsproblem umgangen. Solche Verbindungen sind jedoch bisher nur mittels komplizierter und verlustreicher vielstufiger Synthesen zugänglich. Daher konnten bisher auch noch keine umfangreicheren Untersuchungen zum Hybridisierungsverhalten, vor allem in Zellkulturen durchgeführt werden.

Eine andere Klasse chemisch modifizierter Oligonucleotide sind solche mit nichtionischen, d.h. neutralen Internucleosidbindungen. Zu dieser Art modifizierter Struktur gehören Oligonucleotide mit Phosphotriester- oder Methylphosphonat-Internucleosidbindungen (Oligodeoxynucleotides, Antisense Inhibitors of Gene Expression, J.S. Cohen, ed. Macmillian Press, 1989, S. 79ff).

Beiden Verbindungsklassen ist gemeinsam, daß der ionisierbare Phosphatrest durch eine ungeladene Gruppe ersetzt ist. Durch die Einführung einer solchen Verknüpfung werden diese Oligonucleotidanalogen resistent gegen Nucleasen, haben jedoch den Nachteil der schlechten Löslichkeit in wäßrigem Medium.

Oligonucleotide mit neutral/achiraler Internucleosidbindung konnten bisher nur gewonnen werden, indem das Phosphoratom der Internucleosidbindung durch ein anderes Zentralatom ersetzt wurde. Der Phosphorsäureesterbindung ähnlich ist eine Siloxan-Verknüpfung. Oligonucleotide mit Siloxan-Internucleosidbindungen (K.K. Ogilvie, J.F. Cormier, Tetrahedron Lett. 26, 4159 (1985), H. Seliger, G. Feger, Nucl. Nucl. 6 (182), 483-484 (1987)) wurden synthetisiert und sind stabil gegen Nucleasen.

Oligonucleotide mit Carbonat-, Acetal- oder Carboxamid-Internucleosidverknüpfungen sind aus M. Matteucci, Tetrahedron Lett. 31, 2385-2388 (1990), J.R. Tittensor, J. Chem. Soc. C. 1971, S. 2656 und J. Coull et al., Tetrahedron Lett. 28, 745 (1987) bekannt.

Nucleaseresistente Oligonucleotidanaloge konnten auch ohne Veränderung der Phosphodiester-internucleotidbindung erzeugt werden, indem Zuckerreste veränderter Konfiguration eingesetzt wurden. Dies geschah durch die Synthese von Oligonucleotidanalogen, bei denen die Nucleobasen $\alpha$-glykosidisch angebunden sind (Oligodeoxynucleotides, Antisense Inhibitors of Gene Expression, J.S. Cohen, ed. Macmillan Press, 1989, S. 119ff). Solche Oligonucleotidanalogen sind jedoch präparativ schwer zugänglich, da sie von Zucker und Base ausgehend aufgebaut werden müssen. Zudem zeigen sie, was den Richtungssinn der Hybridisierung angeht, zum Teil ein anormales Verhalten.

Gegenstand der Erfindung sind Oligonucleotide der Formeln I oder II

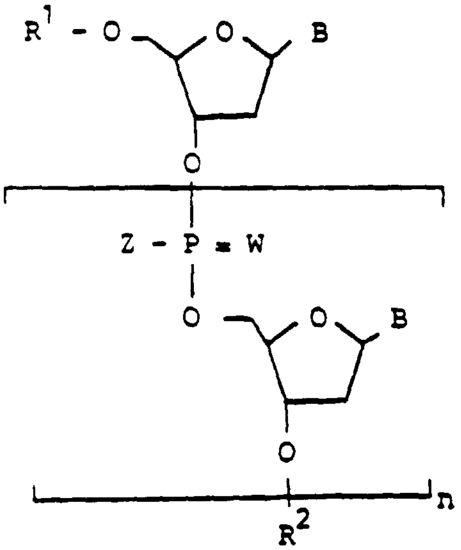

(I)

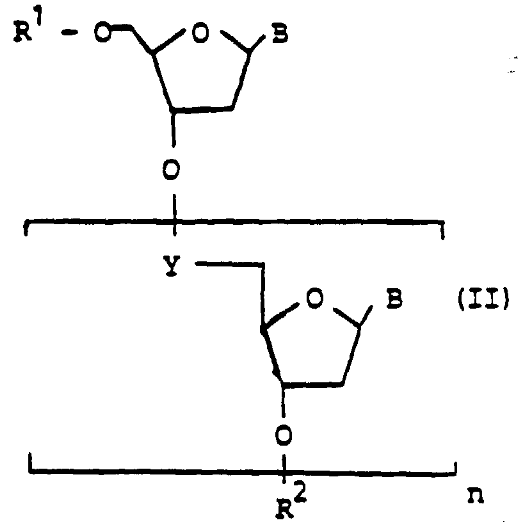

(II)

worin

R[1] Wasserstoff oder einen Rest der Formel III bedeutet

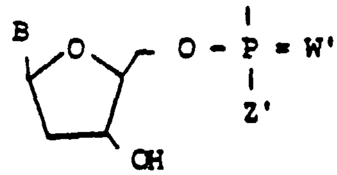

(III)

$R^2$ Wasserstoff oder einen Rest der Formel IV bedeutet,

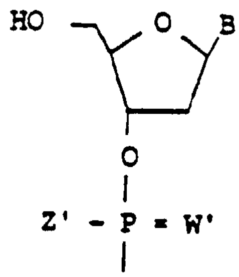

(IV)

wobei jedoch mindestens einer der Reste $R^1$ oder $R^2$ für einen Rest der Formel III oder IV steht;

B für eine Base, wie beispielsweise natürliche Basen wie Adenin, Thymin, Cytosin, Guanin oder unnatürliche Basen, wie beispielsweise Purin, 2,6-Diaminopurin, 7-Deazaadenin, 7-Deazaguanin, $N^4$, $N^4$-Ethanocytosin bzw. deren Prodrugformen steht;

W und W'unabhängig voneinander Sauerstoff oder Schwefel bedeuten;

Z und Z'unabhängig voneinander $O^-$; $S^-$; $C_1$-$C_{18}$-Alkoxy, bevorzugt $C_1$-$C_8$-Alkoxy, besonders bevorzugt $C_1$-$C_3$-Alkoxy, insbesondere Methoxy; $C_1$-$C_{18}$-Alkyl, bevorzugt $C_1$-$C_8$-Alkyl, besonders bevorzugt $C_1$-$C_3$-Alkyl, insbesondere Methyl; $NHR^3$, mit $R^3$ = vorzugsweise $C_1$-$C_{18}$-Alkyl, besonders bevorzugt $C_1$-$C_8$-Alkyl, insbesondere $C_1$-$C_8$-Alkyl, oder $C_1$-$C_4$-Alkoxy-$C_1$-$C_6$-alkyl, bevorzugt Methoxyethyl; $NR^3R^4$, worin $R^3$ wie oben definiert ist und $R^4$ bevorzugt $C_1$-$C_{18}$-Alkyl, besonders bevorzugt $C_1$-$C_8$-Alkyl, insbesondere $C_1$-$C_4$-Alkyl bedeutet oder worin $R^3$ und $R^4$ zusammen mit dem sie tragenden Stickstoffatom einen 5-6-gliedrigen heterozyklischen Ring bedeutet, der zusätzlich ein weiteres Heteroatom aus der Reihe O, S, N enthalten kann, wie z.B. Morpholin;

Y einen Rest aus der Reihe O-Si(R)$_2$, $OCH_2$, C(O)NR oder $OCH_2$-C(O) bedeutet, worin R für $C_1$-$C_6$-Alkyl, Aryl oder $C_5$ bzw. $C_6$ Cycloalkyl steht; und

n eine ganze Zahl von 5-60, bevorzugt 10-40 und insbesondere bevorzugt 15-25 bedeutet,

sowie deren physiologisch verträglichen Salze.

Unter Aryl soll in diesem Zusammenhang verstanden werden z.B. Phenyl, Phenyl substituiert (1-3 fach) mit $C_1$-$C_6$-Alkyl, $C_1$-$C_6$-Alkoxy und/oder Halogen.

Bevorzugt sind die Oligonucleotide der Formel I.

Ferner sind Oligonucleotide der Formel I bevorzugt, worin $R^2$ für einen Rest der Formel IV steht und $R^1$ Wasserstoff bedeutet; $R^1$ bzw. $R^2$ für einen Rest der Formeln III bzw. IV steht; oder $R^2$ Wasserstoff bedeutet und $R^1$ für einen Rest der Formel III steht, wobei entweder W oder Z im letzten Fall nicht Sauerstoff bedeuten.

Insbesondere seien ferner Oligonucleotide der Formel I genannt, worin W für Sauerstoff steht oder Z und W beide für Sauerstoff stehen.

Ganz besonders bevorzugt sind Oligonucleotide der Formel I, worin $R^2$ für einen Rest der Formel IV steht und $R^1$ Wasserstoff bedeutet.

Ferner seien Oligonucleotide der Formeln I und II genannt, die zusätzlich durch Gruppen substituiert sind, die die

intrazelluläre Aufnahme begünstigen, die in vitro oder in vivo als Reportergruppen dienen, und/oder Gruppen, die bei der Hybridisierung des Oligonucleotids an biologische DNA oder RNA diese DNA- oder RNA Moleküle unter Bindung oder Spaltung angreifen.

Beispielhaft für Gruppen, die die intrazelluläre Aufnahme begünstigen, sind lipophile Reste wie Alkylreste z.B. mit bis zu 18 C-Atomen oder Cholesteryl, oder Konjugate, die natürliche Carrier-Systeme ausnutzen, wie z.B. Gallensäure oder Peptide für den entsprechenden Rezeptor (z.B. rezeptorvermittelte Endozytose). Beispiele für Reportergruppen sind fluoreszierende Gruppen (z.B. Acridinyl, Dansyl, Fluoresceinyl) oder chemilumineszierende Gruppen wie z.B. Acridiniumester-Gruppen.

Oligonucleotidkonjugate, die an Nucleinsäuren binden und/oder spalten, enthalten Acridin (N. Thuong et al., Tetrahedron Lett. 29, S. 5905, 1988), Psoralen (U. Peiles et al., Nucleic Acid Res. Bd. 17, S. 285, 1989) oder Chlorethylaminoaryl-Konjugate (Oligodeoxynucleotides, Antisense inhibitors of Gene Expression, J.S. Cohen, ed. Mc Millou Press, 1989, S. 173 ff).

Die charackteristische strukturelle Modifikation der enfindungsgemäßen Oligonucleotide besteht darin, daß die Internucleotidverknüpfungen an den beiden Kettenenden verändert sind, d.h. statt biologischer 3'-5'-Verknüpfungen 3'-3'- bzw. 5'-5'-Verknüpfungen sind. Überraschenderweise fanden wir, daß diese minimale Strukturmodifikation genügt, um solche Verbindungen gegen Nuclease-Abbau zu stabilisieren.

Wie nachstehend beschrieben wird, bewirkt die nur geringfügige Strukturmodifikation ein Hybridisierungsverhalten, das fast der biologischen Oligonucleotide gleicht. Hieraus resultiert auch die allgemeine Anwendbarkeit dieser Verbindungen als Inhibitoren der Genexpression in Zellkulturen.

Aus der Literatur ist bisher kein Hinweis auf die Darstellung von Oligonucleotidanalogen mit 3'-3'- und 5'-5'-Internucleosidverknüpfungen an den beiden Kettenenden und ihren Einsatz antisense-Oligonucleotide bekannt. Analogue von Dinucleosidphosphaten, die entweder 3'-3'- oder 5'-5'-Verknüpfungen enthalten, hat man schon seit vielen Jahren als Haupt- (A. Myles, W. Hutzenlaub, G. Reits und W. Pfleiderer, Chem. Ber. 108, 2857-2871 (1975); H. Rokos, A. Myles, W. Hutzenlaub und W. Pfleiderer, Chem. Ber. 108, 2872-2877 (1975); J. Tomasz, Nucl. Nucl. 2(1), 51-61 (1983); M. Nemer, N. Theriault, A. Schifmann und K.K. Ogilvie, Vith International Round Table, Nucleosides, Nucleotides and their biological Applications, La Grande Motte, ed. J.L. Imbach, 94-96 (1984)) bzw. Nebenprodukte (R. Letsinger, K.K. Ogilvie, J. Amer. Chem. Soc. 89, 4801-4802 (1967)) entsprechender Kondensation erhalten und untersucht. Solche Dimeren sind jedoch aufgrund des zu niedrigen Schmelzpunkts grundsätzlich nicht zu stabiler Hybridisierung mit zellulären Nucleinsäuren befähigt.
Oligodesoxynucleotide, die an einem Ende eine 5'-5'-Verknüpfung zu einem Monoribonukleotid haben, wurden aufgebaut, um eine Anheftungsstelle für Reportergruppen bei Gensonden einzuführen (S. Agrawal, C. Christodoulou, M.J. Gait, Nucleic Acids Res 14, 6227-6245 (1986)).
Sie wurden jedoch bisher nicht auf ihr Hybridisierungsverhalten untersucht. Für biophysikalische Untersuchungen hat man selbstkomplementäre Oligonucleotide dargestellt, die in der Mitte des Moleküls eine einzelne 3'-3'- bzw. 5'-5'-Verknüpfung aufweisen (J.H. van de Sande, N.B. Ramsin, M.W. German, W. Elhorst, B.W. Kalisch, E.V. Kitzing, R.T. Pon, R.c. Clegg, T.M. Jovin, Science 241, 551-5557 (1988)).

Die Darstellung von Oligonucleotiden mit terminal invertierter 3'-3'- und 5'-5'-Bindung erfolgt in gleicher Weise wie die Synthese biologischer Oligonucleotide in Lösung oder vorzugsweise an fester Phase, gegebenenfalls unter Zuhilfennahme eines automatischen Synthesegeräts.

Gegenstand der Erfindung ist daher weiterhin ein Verfahren zur Herstellung der Oligonucleotide der Formel I, dadurch gekennzeichnet, daß man

a) eine Nucleotideinheit mit 3'- bzw. 5'-terminaler Phosphor(III)- oder Phosphor(V)-Gruppierungen oder deren aktiviertes Derivat mit einer weiteren Nucleotideinheit mit 3'- bzw. 5'-terminaler freier Hydroxygruppe umsetzt oder
b) das Oligonucleotid durch Fragmente in gleicher Weise aufbaut, in den nach (a) oder (b) erhaltenen Oligonucleotiden gegebenenalls eine oder mehrere zum Schutz anderer Funktionen temporär eingeführte Schutzgruppen abspaltet und die so erhaltenen Oligonucleotide der Formel I gegebenenfalls in ihr physiologisch verträgliches Salz überführt.

Als Ausgangskomponente zur Herstellung von Oligonucleotiden mit terminal invertierter 3'3' Bindung wird für die Festphasensynthese ein Trägerharz eingesetzt, an das das erste Nucleosidmonomere über die 5'-OH-Gruppe angeknüpft ist. Zur Herstellung dieser Komponente verwendet man ein nach literaturbekannten Methoden (T. Atkinson, M Smith in Oligonucleotide Synthesis, M.J. Geit (ed), 35-49 (1984)) dargesteltes Trägerharz, vorzugsweise Kieselgel oder controlled pore glass, das mit Aminogruppen funktionalisiert ist. Es wird mit einem an der Nucleobase und an der 3'-OH Gruppe geschützten Nucleosidderivat umgesetzt, das zuvor ins 5'-p-Nitro-phenylsuccinat umgewandelt worden war. Als Basenschutzgruppen werden vorzugsweise Acylgruppen, z.B. Benzoyl, Isobutyryl oder Phenoxyacetyl, eingesetzt. Die 3'-Position wird vorzugsweise durch die Dimethoxytrityl-Schutzgruppe geschützt, die nach M. D. Matteucci, M.H. Caruthers, Tetrahedron Letters 21 (1980), S. 3243 - 3246, eingeführt werden kann. Der weitere Aufbau der Oligonucleotidkette bis zum vorletzten Kettenglied erfolgt nach literaturbekannten Methoden, vorzugsweise unter Verwendung von an der 5'-OH-Gruppe durch Dimethoxytritylgruppen geschützten Nucleosid-3-phosphorigsäureesteramiden oder Nucleosid-3'-H-phosphonaten. Als letztes Kettenglied wird wieder ein an der 3'-OH-Gruppe, vorzugsweise mit Dimethoxytrityl geschütztes Nucleosid-5'phosphorigsäureesteramid bzw. Nucleosid-H-phosphonat eingesetzt. Die Darstellung einer solchen Oligonucleotidkette mit terminal verdrehten Internucleotidbindungen ist nachfolgend schematisch wiedergegeben. (Phosphoramiditzyklus zur Darstellung von Oligonucleotiden mit 3'-3'- und 5'-5'-Verknüpfungen an den Enden). Die Darstellung von Oligonucleotiden mit 3'-3'- oder 5'-5'-Verknüpfungen erfolgt entsprechend.

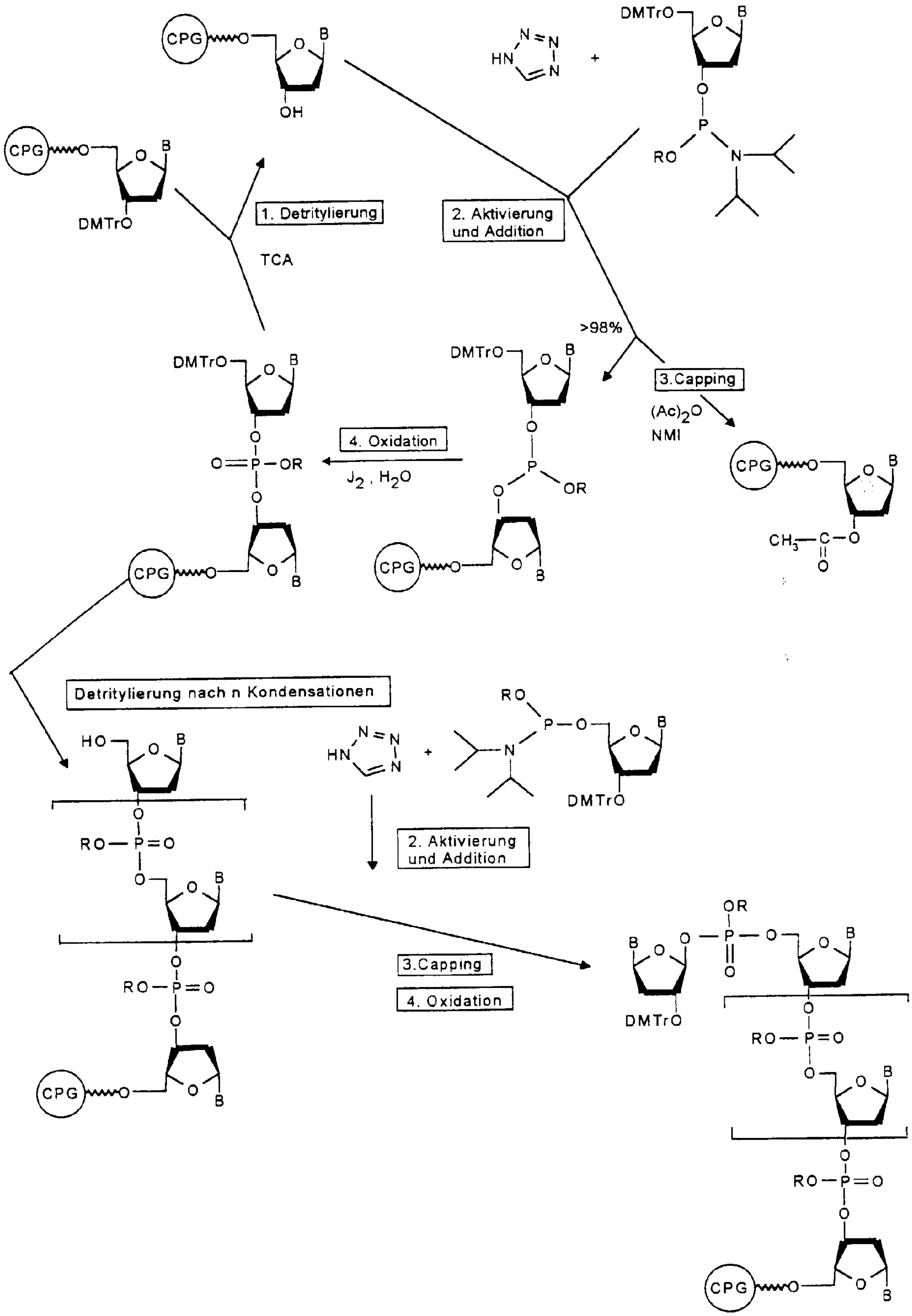


Die Struktur- und Sequenzanalyse erfolgt in der Weise, daß das mit 3'-3'- und 5'-5'-Enden aufgebaute Oligonucleotid zunächst terminal markiert wird. Dies geschieht durch radioaktive Markierung, vorzugsweise mit Hilfe von 5'-$\gamma$-$^{32}$P-ATP/Polynucleotidkinase. Diese radioaktive Markierung erfolgt an der freien 5'-OH-Gruppe, d.h. gegenüber einem Oligonuoleotid mit nur biologischem 3'-5'-Verknüpfungen am umgekehrten Ende der Nucleotidkette. Die weitere Sequenzanalyse erfolgt dann nach literaturbekannten Verfahren, vorzugsweise durch basenspezifische, statistische Spaltung, wie von Maxam und Gilbert beschrieben (A.M. Maxam and W. Gilbert, Methods in Enzymology 65, 499-560 (1980)).

Aufgrund der radioaktiven Markierung am "verkehrten" Molekülende erfolgt nun auch die Sequenzablesung, verglichen mit einem Oligonucleotid mit biologischer 3'-5'-Verknüpfung, im umgekehrten Richtungssinn. Eine ausführliche Testbeschreibung ist in Beispiel 6 angegeben.

Die Oligonucleotide der Formeln I und II finden Anwendung für hybridisierungschemische, auf der Anlagerung an doppel- oder einzelsträngige Nucleinsäuren beruhende Verfahren der Regulation oder Unterdrückung der biologischen Funktion von Nucleinsäuren sowie zur selektiven Unterdrückung der Expression viraler Genomfunktionen und zur Prophylaxe und Therapie von Virusfunktionen, zur Unterdrückung der Onkogenfunktion und zur Therapie von Krebserkrankungen.

Als Maß für die Stabilität in vivo kann das Verhalten eines im Blutserum gelösten erfindungsgemäß aufgebauten Oligonucleotids der Formel I oder II angesehen werden. Der allgemeine Test ist in Beipsiel 7 beschrieben; ein entsprechender in vitro Test mit einer Lösung von Schlangengiftphosphodiesterase in Beispiel 8. Die erfindungsgemäßen Oligonucleotide werden im Gegensatz zu den 3'-5'-Oligonucleotiden viel langsamer abgebaut.

Beispiel 11 demonstriert die Serum-Stabilität von Oligonucleotiden mit erminal invertierter 3'3'-Bindung.

Das Hybridisierungsverhalten ergibt sich aus Modellversuchen, wie in Beispiel 9 beschrieben, in denen eine Reihe von SV40-spezifischen Sequenzen, die erfindungsgemäß mit jeweils einem 3'-3'- und 5'-5'-Ende dargestellt wurden, in der Hybridisierung mit dem entsprechenden Gegenstrang aus SV40-DNA einen Schmelzpunkt zeigen, der nur unwesentlich niedriger liegt als der Schmelzpunkt, der bei der Hybridisierung mit einer entsprechenden Sequenz gemessen wird, die nicht erfindungsgemäß, d.h. ohne 3'-3'- und 5'-5'-Ende, aufgebaut wurde.

Die Wirksamkeit der erfindungsgemäß mit terminalen 3'-3'- bzw. 5'-5'-Verknüpfungen versehenen Oligonucleotide als Inhibitoren der Genexpression ergibt sich aus der Unterdrückung des Wachsstums des Virus SV40. (Beispiel 10) sowie der Inhibition der in vitro Expression des Onkogenproduktes p53 (Beispiel 12).

Beispiel 1

Synthese von 3'-O-DMTr-Desoxyribonucleosid-5'-(N,N-bis-diisopropyl-amino-β-cyanethoxy-)phosphorigsäureesteramid

Das Reaktionsschema - ausgehend von den basengeschützten Nucleosiden - ist nachfolgend dargestellt:

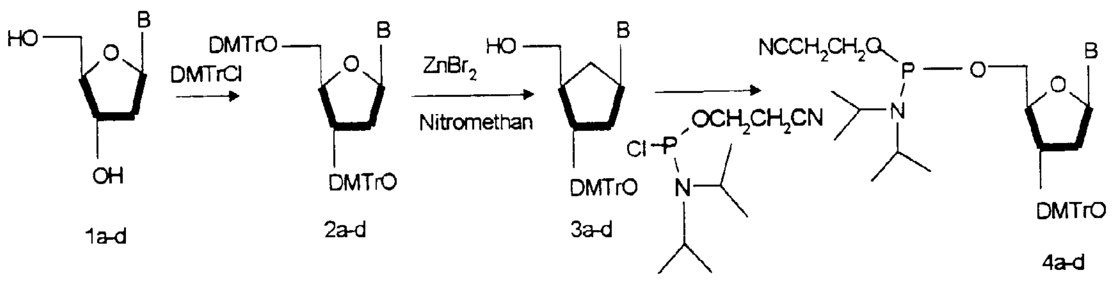


a: B = Thymin
b: B=N$^5$-Benzoyladenin
c: B = N$^4$-Benzoylcytosin
d: B=N$^2$-Isobutyrylguanin

DMTr = 4,4'-Dimethoxytrityl

A) Darstellung der 3',5'-O-Bis-DMTr-Desoxyribonucleoside 2

Ansatz: 6 mMol dT, dA$^{bz}$, dC$^{bz}$ oder dG$^{ibu}$

14,4 mMol DMTr-Cl
14,9 mMol TEA abs.

Das Desoxyribonucleosid 1 wird in 50 ml absolutem Pyridin gelöst und bei 0 °C Triethylamin und Dimethoxytritylchlorid zugegeben. Anschließend läßt man 24 h bei Raumtemperatur rühren, wobei die Umsetzung dünnschichtchromatrographisch (Laufmittel: $CH_2Cl_2$/MeOH 9:1) verfolgt wird. Die Reaktion wird durch Zugabe von Methanol abgebrochen, das Lösungsmittel abgezogen und der Rückstand in Dichlormethan aufgenommen. Die organische Phase wird mehrmals mit 1 M $(NH_4)HCO_3$ - Lösung und mit $H_2O$ gewaschen, über $Na_2SO_4$ getrocknet und einrotiert.
Das 3',5'-ditritylierte Produkt 2 kann durch Säulenchromatographie von überschüssigem Tritanol bzw. 5'-DMTr-dN gereinigt werden (Säulenmaterial: Kieselgel 60H, Elutionsmittel: $CH_2$-$Cl_2$ mit steigendem Methanolgehalt, wobei die ditritylierten Desoxy-nucleoside bei 1-1,5 % MeOH eluiert werden).
Ausbeute: 73-85 % d.Th.

B) Spezifische Abspaltung der 5'-Dimethoxytritylgruppe

Literatur: M.D. Matteucci and M.H. Caruthers, Tetrahedron Lett. 21, 3243-3246 (1980)
Ansatz: 3 mMol 3'-,5'-O-Bis-DMTr-dN 2
15 mMol $ZnBr_2$
200 ml absolutem Nitromethan

Die Lösung von 3'-,5'-O-Bis-DMTr-dN 2 in 100 ml Nitromethan wird bei 0 °C zur Suspension des Zinkbromids in weiteren 100 ml Nitromethan gegeben. Im Falle des geschützten Desoxyadenosins wurde die Reaktion unter Kühlung weitergeführt, um eine Depurinierung zu vermeiden. Die Abspaltung ist bei diesem Nudeosid dennoch schon nach 60 Min. vollständig, wie sich dünnschichtchromatographisch feststellen läßt. Bei Thymidin und Desoxyguanosin ist die Reaktion ebenfalls nach 60 Min. bei Raumtemperatur beendet, während die 5'-Detritylierung von Bis-DMTr-desoxycytidin nur unvollständig verläuft. Hier wurde die Reaktion nach 3 Stunden abgebrochen, um eine Abspaltung auch der 3'-Dimethoxy-tritylgruppe zu vermeiden.

Der Abbruch der Reaktion erfolgt durch Zugabe von 200 ml 1 M $NH_4$Ac-Lösung; das Produkt 3 wird mit 200 ml $CH_2Cl_2$ extrahiert, die organische Phase nochmals mit ges. NaCl-Lösung und mit $H_2O$ gewaschen, über $Na_2SO_4$ getrocknet und das Lösungsmittel im Vakuum abdestilliert.

Die Hauptmenge an Tritanol kann durch Ausfällen des Rohprodukts in ca. 500 ml n-Hexan bei -15 °C abgetrennt werden. Die Reinigung erfolgt dann chromatographisch über eine Kieselgel 60H-Säule mit Dichlormethan mit steigendem Gehalt an Methanol als Elutionsmittel.
Die $R_f$-Werte der 3'-DMTr-Desoxyribonucleoside unterscheiden sich von den entsprechenden 5'-triylierten Monomeren folgendermaßen (Laufmittel: $CH_2Cl_2$/MeH 24:1):

| | 3'-DMTr-dN | 5'-DMTr-dN |
|---|---|---|
| dT | 0,28 | 0,21 |
| $Da^{bz}$ | 0,55 | 0,29 |
| $dC^{bz}$ | 0,53 | 0,31 |
| $dG^{ibu}$ | 0,32 | 0,14 |

Ausbeuten: 48-65 % d.Th.

C) Darstellung der 3'-O-DMTr-Desoxytribonucleosid-5'-O-(N,N-diisopropylamino-β-cyanoethoxy-)phosphorigsäureesteramide 4

Literatur: H. Köster, Nucleic Acids Res. 12, 4539-4557 (1984)
Ansatz: 1 mMol 3'-O-DMTr-Desoxyribonucleosid 3
1,5 mMol Chloro-N,N-diisopropylamino-β-cyanoethoxy-phosphin
4 mMol Diisopropylamin

Die Phosphitylierungsreaktionen wurden analog der von Köster et al.
beschriebenen Methode zur Darstellung der 5'4%DMTr-Desoxytribonucleosid-3'-O-phosphorigsäureesteramide durchgeführt. Zur Lösung der geschützten Nucleoside in absolutem $CH_2Cl_2$ wurde unter Argon Diisopropylamin, anschließend das Phosphorylierungsmittel zugefügt. Nach 30 Min. wurde die Reaktion durch Zugabe von 30 ml Ethylacetat abgebrochen, die Lösung 3 x mit ges. NaCl-Lösung extahiert, die organische Phase über $Na_2SO_4$ getrocknet und das Lösungsmittel im Vakuum abdestilliert. Das Rohprodukt wurde säulenchromatographisch gereinigt (Säulenmaterial:Kieselgel 60H, Elutionsmittel:
Petrolether/Dichlormethan/Triethylamin 45:45:10).
Ausbeute an 4a: 93 % d.Th.

Beispiel 2

Darstellung von Thymidilyl-thymidin mit einer 3'- 3"-Phosphodiesterverknüpfung

Nachfolgend ist der Reaktionsweg zur Darstellung des Dimerblocks angegeben:

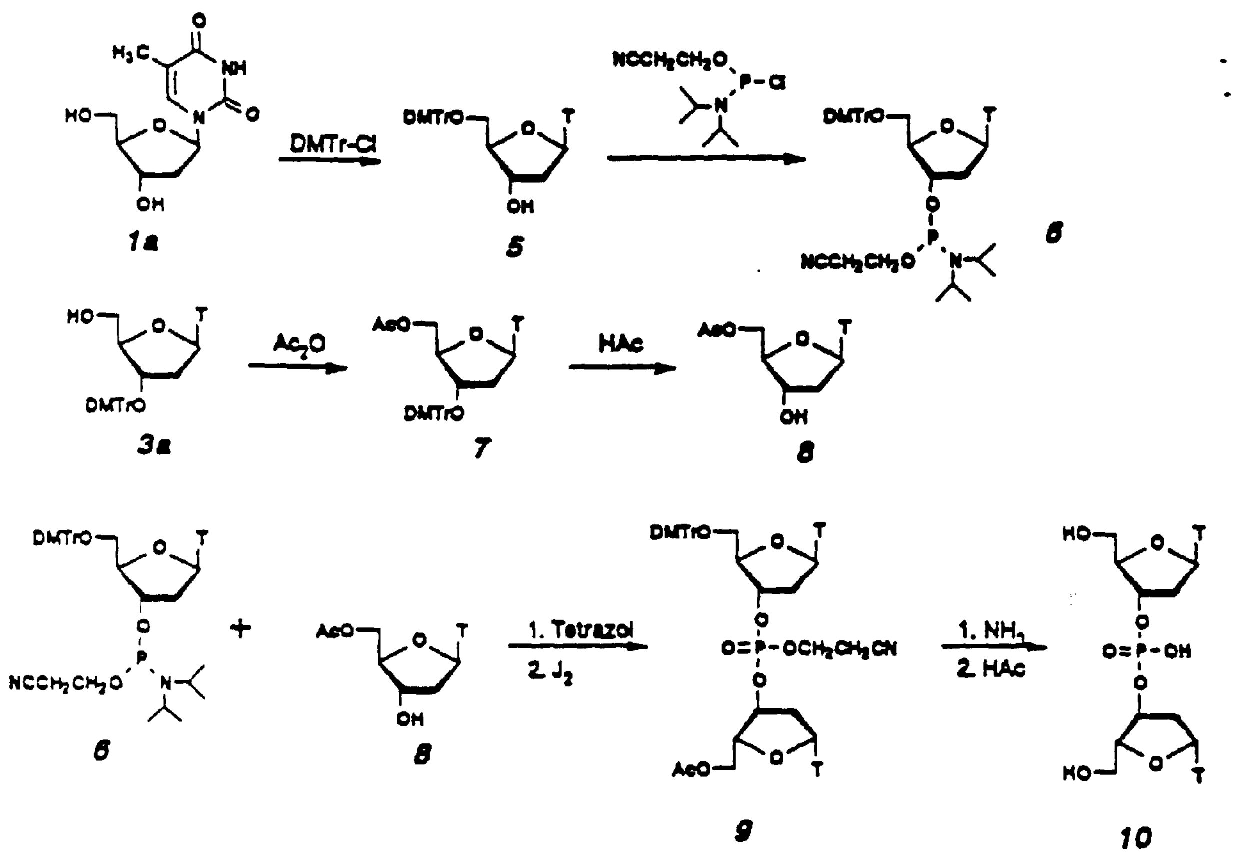


A) Darstellung von 5'-O-Dimethoxytritylthymidin 5

Ansatz: 20 mMol Thymidin (4,84 g)
24 mMol 4,4'-Dimethoxytritylchlorid (8,2 g)
1 mMol Dimethylaminopyridin (122 mg)
28 mMol abs. Triethylamin (3,8 ml)

Die Darstellung von 5 wurde nach der Methode von R.A. Jones (G.S. Ti, B.L. Gaffney and R.A. Jones, H. Amer. Chem. Soc. 104, 1316-1319 (1982)) durchgeführt. Thymidin wurde durch 3maliges Coevaporieren mit jeweils 50 ml absolutem Pyridin getrocknet, in 100 ml Pyridin aufgenommen und unter Eiskühlung 4,4'-DMTr-Cl und DMAP als Katalysator zugegeben. Die Umsetzung wurde dünnschichtchromatographisch kontrolliert (Laufmittel $CH_2Cl_2$/MeOH 9:1); nach 4 Stunden konnte die Reaktion durch Zugabe von 100 ml Wasser abgebrochen werden. Die Lösung wurde 3 x mit Ether extrahiert, die organische Phase getrocknet und einrotiert und der Rückstand durch Umkristallisieren in Benzol gereinigt.
Ausbeute: 9,68 g DMTr-dT (89 %)

B) Darstellung von 5'-O-Dimethoxytritylthymidin-3'-O-(N,N-diisopropylamino-β-cyanoethoxy-)phosphorigsäureesteramid 6

Die Darstellung und Reinigung von 6 erfolgte entsprechend der Synthese der 3'-O-DMTr-5'-O-phosphorigsäureesterstraße 4 (Beispiel 1, C).

C) Darstellung von 5'-O-Acetylthymidin 8

Ansatz: 2,0 mMol 3'-O-DMTr-dT 3a (1,1 g)
0,2 mMol Dimethylaminopyridin (12,2 mg)
4 ml Essigsäureanhydrid
Literatur: A.M. Michelson and A.R. Todd, J. Org. Chem. 1955, 2632-2638 (modifiziert).

Zur Lösung von 3'-O-DMTr-dT und DMAP in 20 ml absolutem Pyridin wurde $Ac_2O$ unter Eiskühlung zugetropft. Nach 3 Stunden Reaktionszeit war die Umsetzung vollständig (Kontrolle über DC; Laufmittel: $CH_2Cl_2$/MeOH 24:1). Das Produkt 7 wurde in 400 ml Eiswasser ausgefällt, der weiße Niederschlag abgesaugt, mit Eiswasser gewaschen und getrocknet.

Ausbeute: 1,02 g 3'-O-DMTr-5'-O-Ac-dT (88%)

Zur Abspaltung der 4,4'-Dimethoxytritylgruppe wurde 7 in 10 ml 80 %iger Essigsäure bei Raumtemperatur gerührt. Durch Zugabe von 100 ml Eiswasser wurde die Reaktion nach 3 Stunden abgebrochen; dabei fiel 4,4'-Dimethoxytritanol als weißer Niederschlag aus. Es wurde abgesaugt und das wäßrige Filtrat einrotiert. Der ölige Rückstand wurde in wenig $CH_2Cl_2$ aufgenommen und in 200 ml n-Hexan bei -15 °C ausgefällt.

Ausbeute: 455 mg 5'-O-Acetylthymidin (94%)

D) Darstellung und Thymidilyl-(3'-3')thymidin 10

Ansatz: 1,0 mMol 5'-O-DMTr-thymidin 3'-O-(N,N-diisopropylamino-,β-cyanoethoxy-)-phosphorigsäureesteramid 6 (705,8 mg)
0,9 mMol 5'-O-Acetylthymidin 8 (253 mg)
2,6 mMol Tetrazol (182 mg)
9,0 mMol $J_2$ (1,14g)

Die Synthese des Dimerblocks 10 konnte nach der von Köster (H. Köster, Nucleic Acids Res. 12, 4539-4557 (1984)) beschriebenen Methode zur Darstellung von 3'-5'-Nucleosiddimeren durchgeführt werden. Die Mischung aus 5'-O-Acetylthymidin und Tetrazol wurde in 30 ml absolutem $CH_3CN$ gelöst und unter Argon zum Phosphorigsäureesteramid gegeben. Der Ansatz von 9 mMol $J_2$ in 30 ml Acetonitril/Pyridin/Wasser (24:5:1) zugefügt und nach weiteren 15 Minuten das überschüssige Jod durch 5 ml einer 40 %igern $H_2SO_3$-Lösung reduziert. Die Lösung wurde dann eingeengt, der Rückstand in $CH_2Cl_2$ aufgenommen, 2 x mit ges. $NaHCO_3$-Lösung gewaschen und das Lösungsmittel dann abdestilliert. Das Rohprodukt konnte säulenchromatographisch gereinigt werden (Säulenmaterial:Kieselgel 60H; Elutionsmittel:Essigester/Dichlor-methan 50:50).

Ausbeute: 665 mg (75 %)

Zur Abspaltung der Acetyl- und β-Cyanoethylgruppe wurde das Dimere 1 Stunde mit 5 ml konz. $NH_3$ bei Raumtemperatur behandelt; die 4,4'-Dimethoxytritylgruppe konnten mit 80 %iger Essigsäure entfernt werden.

Beispiel 3

Darstellung von Thymidilyl-Thymidin mit 5'-5-Phosphodiesterverknüpfung 14

Nachfolgendes Schema zeigt die Darstellung von 14

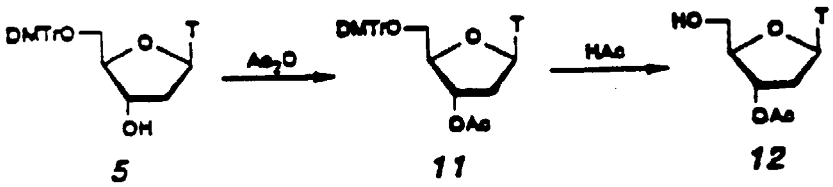


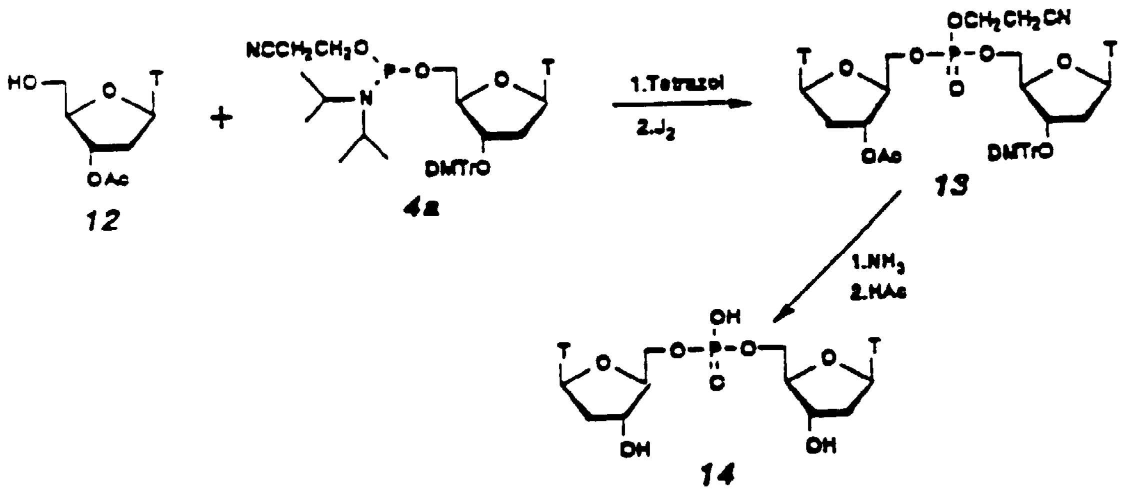


Die einzelnen Reaktionsschritte zur Synthese der Zwischenstufen erfolgten entsprechend den in Beispiel 2 beschriebenen Methoden.

Die Struktur der beiden Dimerblöcke konnte durch FAB-Massenspektrometrie und 1H-Kernresonanzspektroskopie verifiziert werden.

Beispiel 4

Beladung von CPG 10-1400-Trägermaterial mit 3'-O-Dimethoxytrityldesoxyribonucleosid-5'-O-succinat

Die Funktionalisierung des CPG-Trägers mit 3-Aminopropylketten erfolgte nach der Vorschrift von Atkinson und Smith (T. Atkinson, M. Smith in Oligonucleotide Synthesis, M.J. Gait (ed), 35-49 (1984)).

A) Darstellung von 3'-O-DMTr-desoxyribonucleosid-5'-O-succinat 15

Ansatz: 1,0 mMol 3'-O-DMTr-dN 3
0,8 mMol Bernsteinsäureanhydrid(80 mg)
0,5 mMol Dimethylaminopyridin (61 mg)

Die Reaktion von Bernsteinsäureanhydrid mit der 5'-OH-Gruppe der Desoxyribonucleoside wurde jeweils in 5 ml absolutem Pyridin mit DMAP als Katalysator über Nacht bei Raumtemperatur durchgeführt. Nach vollständiger Umsetzung wurde die Lösung eingeengt und das Pyridin durch 3malige azeotrope Destillation mit Toluol entfernt. Der Rückstand wurde in Dichlormethan aufgenommen, mit 10 %iger eiskalter Citronensäurelösung und $H_2O$ gewaschen und die organische Phase im Vakuum einrotiert. Das Rohprodukt wurde in ca. 3 ml Toluol gelöst und in 200 ml n-Hexan ausgefällt.

Ausbeuten: 79-84 %

B) Darstellung der 3'-O-DMTr-desoxyribonucleosid-5'-O-succinyl-p-nitrophenylester 16 und Trägerbeladung (siehe nachfolgendes Schema)

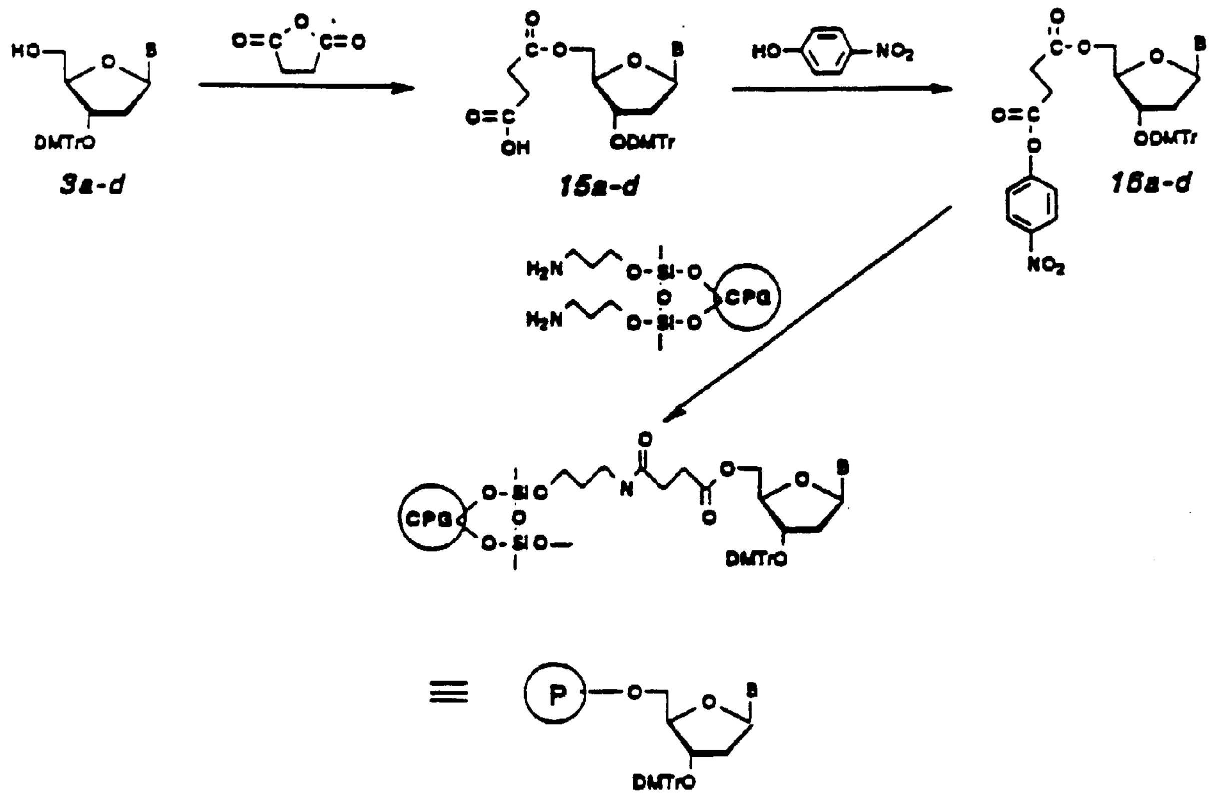

3a-d 15a-d 16a-d

Ansatz:
0,8 mMol 3'-O-DMTr-dN-5'-O-succinat 15
0,8 mMol p-Nitrophenol (112 mg)
2,0 mMol Dicyclohexylcarbodiimid (412 mg)
3 g funtionalisiertes CPG 10-1400

Das geschütze succinylierte Desoxytribonucleosid wurde zu einer Lösung von p-Nitrophenol in 5 ml absolutem Dioxan und 0,2 ml Pyridin gegeben und anschließend DCCI als Kondensationsmittel zugefügt. Bereits nach kurzer Zeit fiel Dicyclohexylharnstoff aus und nach 3 Stunden zeigte das DC ($CH_2Cl_2$/MeOH 9:1) eine vollständige Umsetzung an. Der Niederschlag wurde unter Argon abgesaugt und das Filtrat direkt zu einer Suspension des funktionalisierten Trägermaterials in 15 ml absolutem DMF gegeben. Man fügte 0,8 ml Triethylamin hinzu und schüttelte den Ansatz über Nacht. Der beladene Träger wurde dann abgesaugt, mit Methanol und Ether gewaschen und im Exsikkator getrocknet.

Die Beladung des Trägers wurde spektrometrisch bestimmt. Durch Behandeln einer Trägerprobe (ca. 2 mg) mit 10 ml einer 0,1 N p-Toluolsulfonsäurelösung in Acetonitril wird das 4,4'-Dimethoxytritylkation abgespalten und durch Messung der Absorption der Lösung bei 498 nm läßt sich der Gleichung

$$\frac{OD_{498}/\text{ml x 10 ml x 14,3 (Konst.)}}{\text{mg Träger}}$$

die Trägerbeladung in µMol/g berechnen.

Ergebnisse:
3'-DMTr-dT-Träger: 23 µMol/g
3'-DMTr-dG$^{ibu}$-Träger: 21 µMol/g
3'-DMTr-dA$^{bz}$-Träger: 21 µMol/g
3'-DMTr-dC$^{bz}$-Träger: 15 µMol/g

Zur Blockierung nicht umgesetzer Aminogruppen wurde der beladene Träger mit einer Lösung von 1 ml Essigsäureanhydrid und 50 mg Dimethylaminopyridin in 15 ml absolutem Pyridin 1 Stunde bei Raumtemperatur geschüttelt, anschließend abgesaugt, mit Methanol und Ether gewaschen und getrocknet.

Beispiel 5

a) Synthese von Oligonucleotiden mit teminaler 3'-3'- und 5'-5'-Verknüpfung

Die Synthesen wurden in einem DNA-Synthesizer der Firma Applied Biosystems, Forster City, USA, Modell 381A, unter Verwendung des 0,2 µMol-Standardprogramms für alle Kondensationsschritte durchgeführt.

Der Synthesezyklus ist oben gezeigt (S. 11). Als Trägermaterial diente das in Beispiel 4 beschriebene CPG 10-1400. Die Kondensationen wurden mit den üblichen Nucleosid-3'-phosphorigsäureestern durchgeführt; lediglich im letzten Reaktionszyklus wurden die in Beispiel 1 beschriebenen 3'-O-DMTr-Nucleosid-5'-O-(N,N-diisopropylamino-β-cyano-ethoxy-)phosphorigsäureesteramide eingesetzt. Nach Abspaltung des Oligonucleotids vom Träger mit konz. $NH_3$ und Entfernen aller Phosphat- und Basenschutzgruppen durch Erwärmen der Ammoniaklösung auf 60 °C über Nacht wurde die Probe durch Ausfällen in Ethanol entsalzt und die Zielsequenz durch Polyacrylamidgelelektrophorese von kürzeren Fragmenten gereinigt.

Für unsere Untersuchungen synthetisierten wir ein Eicosathymidylat mit nur 3'-5'-Verknüpfungen sowie eines mit terminalen 3'-3'- und 5'-5'-Bindungen. Außerdem wählten wir Sequenzen aus dem SV40-Genom. Folgende Oligonucleotide wurden synthetisiert:

$dT_{20}$;

$dT_{20}$ (3'-3',5'-5');

SV40TS17: 17-mer, Sense-Sequenz aus dem SV40-Genom an den Positionen 5159-5176;

**5'-AGC TTT GCA AAG ATG GA-3'**

SV40TAS17: 17-mer, Antisense-Sequenz zu SV40TS17;

**5'-TCC ATC TTT GCA AAG CT-3'**

SV40TAS17(3'-3',5'-5'):17-mer, Antisense-Sequenz zu SV40TS17 mit einer 3'-3'-bzw. 5'-5'-Verknüpfung an den Enden;

**3'-T(5'-5')CC ATC TTT GCA AAG C(3'-3')T-5'**

SV40TS35: 35-mer, Sense-Sequenz aus dem SV40-Genom an den Positionen 5142-5176;

**5'-AGC TTT GCA AAG ATG GAT AAA GTT TTA AAC AGA AG-3'**

SV40TAS35: 35-mer, Antisense-Sequenz zu SV40TS35;

**5'-TCT CTG TTT AAA ACT TTA TCC ATC TTT GCA AAG CT-3'**

SV40TAS35(3'-3',5'-5'):35-mer, Antisense-Sequenz zu SV40TS35 mit einer 3'-3'-bzw. 5'-5'-Verknüpfung an den Enden;

**3'-T(5'-5')CT CTG TTT AAA ACT TTA TCC ATC TTT GCA AAGC(3'-3')T-5'**

b) Synthese von Oligonucleotiden mit terminaler 3'-3'-Verknüpfung

Als Trägermaterial diente das in Beispiel 4 beschriebene CPG 10-1400. Die Kondensationen wurden mit den üblichen Nucleosid-3'-phosphorigsäureestern durchgeführt.
Nach Abspaltung des Oligonucleotids vom Träger mit konz. $NH_3$ und Entfernen aller Phosphat- und Basenschutzgruppen durch Erwärmen der Ammoniaklösung auf 60 °C über Nacht wurde die Probe durch Ausfällen in Ethanol entsalzt und die Zielsequenz durch Polyacrylamidgelelektrophoese von kürzen Fragmenten gereinigt.

SV40TAS17(3'-3'): 17-mer, Antisense Sequenz zu SV40TS17 mit einer 3'-3'-5'-5'-Verknüpfung am Ende;

**3'-TCC ATC TTT GCA AAG C(3'-3')T-5'**

c) Synthese von Oligonucleotiden mit terminaler 5'-Verknüpfung und 2 terminalen Thiophosphatresten

Als Trägermaterial diente kommerziell erhältliches CPG-material, das 5'-O-Dimethoxytritylthymidin über die 3'-Hydroxygruppe gebunden hält. Die Kondensationen wurden mit den üblichen Nucleosid-3'-phosphorigsäurtestern durchgeführt; lediglich im letzten Reaktionszyklus wurden die in Beispiel 1beschriebenen 3'-O-DMTr-Nucleosid-5'-O-(N,N-diisopropylamino-β-cyano-ethoxy)phosphorigsäureesteramide eingesetzt.

Bei den ersten beiden Reaktionszyklen wird anstelle der üblichen Jod-Oxidation eine Oxidation mit elementarem Schwefel (W. Stec et al. J.A.C.S. 106, S. 6077, 1984) durchgeführt.
Nach Abspaltung des Oligonucleotids vom Träger mit konz. $NH_3$ und Entfernen aller Phosphat- und Basenschutzgruppen durch Erwärmen der Ammoniaklösung auf 60 °C über Nacht wurde die Probe durch Ausfallen in Ethanol entsalzt und die Zielsequenz durch Polyacrylamidgelektrophorese von kürzeren Fragmenten gereinigt.

Folgendes Oligonucleotid wurde synthetisiert:

SV40TAS17(5'-5'): 17-mer, Antisense-Sequenz zu SV40TS17 mit einer 5'-5'-Verknüpfung am 5'-Ende und zwei Thiophosphatinternucleotid-Bindungen am 3'-Ende

**3'-T(5'-5')CC ATC TTT GCA AAG($P_s$) C($P_s$)T-3'**

Beispiel 6

Analyse der Struktur und Sequenz eines Oligonucleotids mit terminaler 3'-3'- und 5'-5'-Verknüpfung

Die Sequenzierung der Oligonucleotide erfolgte nach der Methode von Maxam und Gilbert (A.M. Maxam and W. Gilbert, Methods in Enzymology 65, 499-560(1980)). Jeweils 100 pMol der Proben wurden in Gegenwart von ($\gamma$-$^{32}$P)-ATP/T4-Polynucleotidkinase an der 5'-OH-Gruppe radioaktiv markiert und anschließend folgende basenspezifische Reaktionen durchgeführt:

| | |
|---|---|
| (A+G) - Reaktion: | Protonierung der Basen durch Ameisensäure |
| G - Reaktion: | Umsetzung mit Dimethylsulfat |
| (T+C) - Reaktion: | Hydrazinolyse |
| C - Reaktion: | Reaktion mit Hydrazin in Gegenwart von 5 M NaCl |

Durch Behandlung mit 1 M Piperidin bei 95 °C wurden die Oligonucleotidketten an den modifizierten Stellen gespalten; die Bruchstücke konnten durch Polyacrylamidgelektrophorese (20 % Acrylamid, 7M Harnstoff) aufgetrennt und durch Autoradiographie detektiert werden. Abbildung 1 zeigt das Autoradiogramm der Sequenzierung von SV40TS17, SV40TAS17 und SV40TAS17(3'-3',5'-5') (Beschreibung der Oligonucleotide siehe Beispiel 5). Da die Ketten an der 5'-OH-Gruppe markiert wurden, kann die Basenabfolge bei nur 3'-5'-Verknüpfungen in 3'-5'-Richtung abgelesen werden. Infolge des 3'-3'-Endes hat das Oligonucleotid SV40TAS17(3'-3'-5'-5') jedoch die radioaktiv markierte 5'-OH-Gruppe an seinem 3'-Terminus; die Leserichtung der Sequenz wird dadurch umgedreht (Abb.1). Dies ist auch ein Beweis für die 5'-5'-Phosphodiesterbindung am 5'-Terminus der Kette mit einer freien 3'-OH-Gruppe, die durch das Enzym Polynucleotidkinase nicht phosphoryliert werden kann.

Beispiel 7

Prüfung der Stabilität eines mit 3'-3' und 5'-5'-Enden versehenen Oligonucleotids im Blutserumtest

A) Kinasierung eines Eicosathymidylats mit 3'-3' und 5'-5'-Enden

Literatur: T.Mizuno, M.-Y. Chou and M. Inouya, Proc. Natl. Acad. Sci. USA 81, 1966-1970 (1984)

Ansatz 1 µlOligonucleotid (0,01 $OD_{260}$)
1 µl 10 x Kinase-Puffer
1 µl T4-Polynukleotidkinase
1 µl $y$-$^{32}$P-dATP) (spez. Aktivtät: 6000 Ci/mMol)
6 µl $H_2O$

Der Ansatz wird 30 min bei 37 °C inkubiert, anschließend durch Zugabe von 90 µl $H_2O$ abgebrochen und über eine ®Sephadex G 50 Säule fraktioniert. Die Produktfraktionen werden vereinigt und lyophilisiert.

b) Blutserumtest

Literatur: S.M. Heywood, Nucleic Acids Res. 14, 6771-6772 (1986)
Ansatz: 0,01 $OD_{260}$ radioaktiv phosphoryliertes Oligonucleotid für die Referenz 0,01 $OD_{260}$ $T_{20}$, für das Experiment 0,01 $OD_{260}$ $T_{20}$ mit 3'-3'- und 5'-5' verknüpften Enden
50 µl frisches menschliches Serum

Das Serum wird zur jeweiligen Probe gegeben und kurz von Hand geschüttelt. Dann werden sofort 3 µl für den O-Wert abpipettiert und in 3µl Formamid Loading Buffer gegeben, um die Enzymaktivität abzubrechen. Anschließend werden die Proben bei 37 °C inkubiert.
Für die Kinetik werden nach gewissen Zeitabständen 3 µl abgenommen und wie oben beschrieben abgebrochen.

Referenz: 5 Min, 8 Min, 11 Min, 15 Min, 30 Min
Experiment: 5 Min, 8 Min, 11 Min, 15 Min, 30 Min, 90 Min

Die Proben werden auf 20 %iges Polyacrylamidgel aufgegeben und über Gelelektrophorese getrennt und anschließend autoradiographiert.

Autoradiogramm der Spaltung mit frischem menschlichen Serum von links (siehe Abb. 2):

Referenz: 0-Wert, 5 Min, 8 Min, 11 Min, 15 Min, 30 Min
Experiment: 0-Wert, 5 Min, 8 Min, 11 Min, 15 Min, 30 Min, 90 Min

Schon nach 5 Min zeigt sich, daß der Abbau des natürlichen Oligonucleotids begonnen hat. Die Spaltung nimmt die zunehmende Zeit zu. Das modifizierte Oligonucleotid liegt auch nach 90 minütiger inkubation praktisch unverändert vor. Die schwachen Banden, die bei allen Werten der Kinetik zu sehen sind resultieren aus der Aktivität der Endonukleasen, die im Serum vorhanden sind. Diese Spaltung ist für eine therapeutische Anwendung dieser Oligonucleotide wichtig und wünschenswert, da sie den langsamen Abbau der Wirksubstanz garantierten und es somit nicht zu einer Akkumulation dieser Substanzen im Körper kommen kann.

Beispiel 8:

Untersuchung des Verhaltens von Oligonucleotiden mit terminaler 3'-3'- und 5'-5'-Verknüpfung gegen Schlangengift-Phosphodiesterase

A) Kinasierung eines Eicosathymidylats mit 3'-3'- und 5'-5'-Enden

Wie in Beispiel 7 beschrieben.

B) Hydrolyse eines Eicosathymidylats mit 3'-3' und 5'-5'-Enden mit Hilfe von Schlangengiftphsophodiesterase

Literatur: A. Hirashima, S. Sawaki, Y. Inokuchi and M. Inouye, PNAS USA 83, 7726-7730 (1986)

Ansatz: 5 µlRNA-Carrier
50 µl SQ-Puffer
1 µl SVpdE (1,5 u/µl)

Die radioaktiv phosphorylierte Probe (Referenz: $T_{20}$, Experiment: $T_{20}$ mit terminaler 3'-3'- und 5'-5'-Verknüpfung) wird lyophilisiert, dazu werden der RNA-Carrier und der SQ-Puffer gegeben. Von dieser Mischung werden für den 0-Wert je 5 µl abpipettiert, der Rest der Lösung wird mit Enzym versetzt und bei 37 °C inkubiert. Für die Kinetik der Referenz werden nach 5 Min, nach 15 Min und nach 30 Min je 5 µl entnommen, für die Kinetik des Eicosathymidylats mit 3'-3' 5'-5'-Enden werden nach 5 Min, 30 Min, 45 Min, 60 Min und 90 Min je 5 µl entnommen. Zur Desaktivierung des Enzyms werden die Proben direkt nach der Entnahme im Wasserbad 2 Min auf 95 °C erhitzt. Die Proben werden lyophilisiert und auf ein analytisches 20 %iges Polyacrylamidgel aufgetragen. Nach der geleketrolytischen Auftrennung wird autoradiographiert.

Autoradiogramm der SVpdE-Spaltung (siehe Abb. 3)
von links: $T_{20}$ (Referenz), $T_{20}$ mit den terminalen 3'-3'- und 5'-5'-Verknüpfungen, Kinetik des Experiments: nach 5 Min, 15 Min, 30 Min, 45 Min, 60 Min, 90 Min, Mix aus 0 Min/15Min, Mix aus 0 Min/30 Min., Kinetik der Referenz: nach 5 Min, 15 Min, 30 Min, 45 Min.

Die 5'-5'-Verknüpfung wird, wie schon beschrieben, sofort gespalten. Daß die weitere Spaltung im Vergleich zur Referenz nur noch sehr langsam vor sich geht, ist ein deutlicher Strukturhinweis. Nach Spaltung der 5'-5'-Phosphordiesterbindung ist ein Molekül entstanden, das an der Spaltungsstelle eine 5'-Hydroxylfunktion trägt. Am anderen Terminus ist das Molekül 5'-phosphoryliert. Beide Termini sind für die Schlangengiftphosphodiesterease unangreifbar. Der langsame - aber sehr deutliche - weitere Abbau ist wahrscheinlich auf die vom Hersteller (J.G. Izant and H. Weintraub, Cell 36, 1007-1015 (1984)) beschriebene Beimengung einer einzelstrangspezifischen Endonuklease zurückzuführen, die supercoiled PM2 DNA in offene Formen umwandelt. Schön abzulesen ist die Länge der Sequenz. Es sind jedoch nur 19 Banden abzulesen, da beim ersten Wert der Kinetik, nach 5 Min, die 5'-5'-Phosphodiesterbindung bereits gespalten ist.

Beispiel 9:

Untersuchungen zum Hybridisierungsverhalten von mit terminalen 3'-3'- und 5'-5'-Enden versehenen Oligonucleotiden

Zur Untersuchung des Hybridisierungsverhaltens wurden Schmelzkurven aufgenommen jeweils äquimolare Mengen (5,23 nMol) an SV40TS17 und SV40TAS17(3'-3',5'-5') (Beschreibung der Oligonucleotide siehe Beispiel 5) wurden in 1 ml eines 10 nM Na-cacodylat/100 mM NaCl-Puffers, pH 7,0 gelöst, auf 70 °C erhitzt und der Verlauf der Renaturierung beim langsamen Abkühlen (1 grd/min) durch Messung der Absorption der Lösung ermittelt. Zur Aufnahme der Referenzkurve wurden die gleichen Mengen an SV40TAS17 verwendet. Analog wurden jeweils 4,75 nMol SV40TS35 und SV40TAS35 bzw. SV40TS35 und SV40TAS35(3'-3',5'-5') in dem Puffer gemischt, auf 90 °C erhitzt und die Absorption beim Abkühlen gemessen. Es wurden Schmelzkurven ermittelt, die folgende $T_m$-Werte ergaben:

| | |
|---|---|
| SV40TS17 · SV49TAS17 | 54,1 °C |
| SV40TS17 · SV40TAS17(3'-3',5'-5') | 53,3 °C |
| SV40TS35 · SV40TAS35 | 65,5 °C |
| SV40TS35 · SV40TAS35(3'-3',5'-5') | 64,2 °C |

Die Schmelztemperaturen wurde also durch die abiologischen Verknüpfungen nur um 0,6 ° beim 17mer bzw. 1,3°C beim 35mer erniedrigt.

Beispiel 10

Inhibierung der Biosynthese von SV40 T-Antigen durch Oligonucleotide mit terminaler 3'-3'- und 5'-5'-Verknüpfung

A) Zellkultur

COS1-Zellen wurden in Dulbecco's modifiziertem Eagle Medium (DMEM) mit 10 % fötalem Kälberserum gezüchtet.

Die Gewebekulturschalen wurden bei 37 °C und 7,5 °C $CO_2$ inkubiert.

B) Radioaktive Markierung und Zellaufschluß

Etwa 4 x $10^4$ Zellen wurden als Monolayer in Gewebekulturschalten gezogen. Der konfluente Zellrasen wurde dreimal mit Methionin-freiem DMEM gewaschen und anschließend mit 100 µCi $^{35}$-S-Methionin markiert. Für die Antisense - Modulationsexperimente wurden die Zellen gleichzeitig mit dem Oligonucleotid SV40TAS17(3'-3',5'-5') und radioaktivem Methionin 30 Min. bei 37 °C inkubiert. Anschließend wurden die Zellen zweimal mit DMEM, das 10 % fötales Kälberserum und nicht markiertes Methionin enthielt, gewaschen und weitere 30 Min. in diesem Medium belassen. Zum Aufschluß wurden die Zellen mit eiskaltem Phosphat-gepuffertem Saline (PBS) behandelt, von den Schalen abgeschabt und 2 Min. bei 400 g zentrifugiert. Das Zellpellet wurde dann mit 0,4 ml Aufschlußpuffer (0,5 % Nonidet P40, 100 mM Tris-HCl, pH 9,0, 0,1 M NaCl) pro 3 x $10^6$ Zellen 45 Min. auf Eis lysiert. Um Proteolyse zu vermeiden, wurden dem Aufschlußpuffer 1 % Trasylol und Phenylmethylsulfonylfluorid bis zur Endkonzentration 0,25 mg/ml zugefügt. Das Lysat wurde dann 30 Min. bei 40 °C in der Ultrazentrifuge (105 000 g) klarzentrifugiert. Ein Aliqout von 10 µl wurde entnommen und der Gehalt an Protein nach der Methode von Lowry gemessen. Zur Immunpräzipitation wurden gleiche Mengen an Gesamtprotein eingesetzt.

C) Immunpräzipitation

Sowohl für die Immunpräzipitation von SV40 Wildtyp-T-Antigen als auch von dem im Zytoplasma lokalisierten Mutanten-T-Antigen wurde der monoklonale Antikörper PAb108 verwendet. Der Antikörper wurde durch Chromatographie an Protein A-Sepharose vom Hybridoma-Überstand gereinigt.
Der Zellextrakt wurde mit 100 µl einer 10 %igen Suspension von hitze-inaktivierten und mit Formaldehyd fixierten Bakterien des Stammes Staphylokokkus aureus (Cowan 1) über Nacht bei 4 °C vorpräzipitiert. Die Bakterien wurden dann abzentrifugiert, der Überstand mindestens 2 Stunden lang bei 4 °C mit dem monoklonalen Antikörper inkubiert, und durch Zugabe von S.aureus die hochspezifischen Immunkomplexe gebildet. Dieser Vorgang wurde bis zu 3 mal wiederholt, um eine vollständige Präzipitation zu gewährleisten.
Anschließend wurden die Immunpräzipitate gewaschen: dreimal mit 50 mM Tris-HCl, pH 8,0, 500 mM UCl, 1 mM DTT, 1mM EDTA, 1 % Trasylol; zweimal mit 50 mM Tris-HCl, pH 7,4, 0,15 M NaCl, 5 mM EDTA, 1 % Nonidet P40 und einmal mit 50 mM $NH_4HCO_3$. Sie wurden dann 45 Min. bei 4 °C mit 200 µl Elutionspuffer (50 mM $NH_4HCO_3$, 1 % SDS, 1 % β-Mercaptoethanol) eluiert, lyophylisiert, durch 10-minütiges Kochen in 20 µl Probenpuffer (65 mM Tris-HCl, pH 6,8, 5 & β-Mercaptoethanol, 1 % Glycerin, 0,01 % Bromphenolbau) gelöst und auf ein 3 %iges Polyacrylamidgel (10 % SDS) geladen. Die Proteine wurden durch diskontinuierliche Gelelektrophorese aufgetrennt; die Banden konnten durch Fluorographie auf Röntgenfilm (Kodak X-AR) lokalisiert werden.
Mit Oligonucleotiden, die erfindungsgemäß so dargestellt wurden, daß sie den Translationsstart für das T-Antigen überspannen, wurde bei Anwendung in einer extrazellulären Konzentration von 30 µmolar eine 70 %ige Inhibierung des Virus-Wachstums registriert. Dies steht im Gegensatz zu Wirksamkeit der entsprechenden Sequenzen, die nicht erfindungsgemäß, d.h. ohne 3'-3'- und 5'-5'-Verknüpfungen, aufgebaut waren. Hier war bei Anwendung einer gleichen Konzentration keine Inhibierung feststellbar.
Abbildung 4 zeigt eine deutliche Inhibierung der T-Ag-Biosynthese bei Behandlung der Zellen mit 30 µM SV40TAS17 (3'-3',5'-5').

Beispiel 11

Vergleich der Serumstabilität von Oligonucleotiden die nur am 3'-Ende bzw. nur am 5'-Ende durch invertierte Phosphodiesterbindungen modifiziert sind

Oligonucleotide werden durch Nuclease bevorzugt von ihrem 3'-Terminus her abgebaut (J.P. Shaw, K. Kent, J. Bird, J. Fishback, B. Froehler, Nucleid Acids Res. 19, 747-750 (1991). Es sollte daher untersucht werden, ob allein eine terminale 3'-3'-Phosphodiesterbindung zur Stabilisierung des Oligonucleotids in Blutserum genügt. Da ein mit 32p markierter Phosphatrest - insbesondere bei einer längeren Behandlung der Probe mit Serum - durch Phosphatasen abgespalten wird, wurde zur Detektion der Oligonucleotide Fluorescein verwendet.

Synthetisiert wurden folgende Sequenzen aus dem Genom von Xenopus levis:

**Xelev 1: 3'-A(5'-5')GC CTC AAA C*AT GTG TGA CG-3'**

**Xelev 2:** **5'-AGC CTC AAA C*AT GTG TGA C(3'-3')G-5'**

Die Synthesen wurden wie in Beispiel 5 beschrieben durchgeführt, wobei für die 10. Kondensation ein basenmodifiziertes Cytidin-phosphorigsäureesteramid C* eingesetzt wurde, das eine nachträgliche kovalente Verknüpfung mit Fluorescein ermöglichte. Der Einbau des Fluorescein erfolgte nach einer Anleitung der Firma Pharmacia (Pharmacia LKB Biotechnology Auto Primer™ Synthesis Kit Instructions (XY-023-00-01). Die modifizierten Oligonucleotide wurden durch Polyacrylamidgelelektrophorese gereinigt.

Serumtest: 10 pMol Oligonucleotid
140 µl frisches Humanserum

Die Proben wurden bei 37 °C inkubiert. Nach 15 Min., 30 Min., 45 Min., 60 Min., 8 Std. und 72 Std. wurden jeweils 20 µl entnommen, 2 x mit Phenol/Chloroform/Isoamylalkohol 25:24:1 extrahiert und die Oligonucleotide mit Ethanol gefällt. Die Spaltprodukte wurden auf einem 16 %igen Polyacrylamidgel im automatischen Sequenzer A.L.F. (Pharmacia, Freiburg) getrennt und detektiert. Wie Abbildung 5 zeigt, weist die nur am 5'-Ende mit einer invertierten Internucleotidbindung versehene Sequenz bereits nach 15 Min. Serumbehandlung Abbauprodukte auf und nach 60 Min. ist sie schon nahezu vollständig abgebaut. Das am 3'-Ende durch 3'-3'-Bindung "geschützte" Oligonucleotid dagegen ist selbst nach 72 Stunden erst teilweise abgebaut.

Beispiel 12

Inhibierung der Genexpression in einem in vitro-Expressionssystem

Um die Inhibierung der Genexpression durch INV-Oligonucleotide zu quantifizieren, untersuchten wir die Wirkung von entsprechenden Antisense-Oligonucleotiden auf die Expression von p53 in einem in vitro System, daß p53 mRNA enthielt (E. Reihsaus, M. Kohler, S. Kraiss, M. Oren, M. Montenarli, Oncogene 5, 137-144 (1990)).

Synthetisiert wurden die Antisense-Sequenz

**$NH_2(CH_2)_6$pTAA TCA GTC GTT GGT CCA CAC CTT(3'-3')T**

und als Vergleichsprobe die entsprechende Sense-Sequenz

**$NH_2(CH_2)_6$pAAA GGT GTG GAC CAA CGA CTG ATT(3'-3')A**

Das Antisense-Oligonucleotid ist gegen den Translationsstart des Onkoproteins p53 gerichtet.
Untersucht wurde die Expression des Proteins

1. ohne Zugabe des Oligonucleotids
2. bei Zugabe von 30 µM Sense-Sequenz
3. bei Zugabe von 1 µM Antisense-Sequenz
4. bei Zugabe von 10 µM Antisense-Sequenz

Zur Quantifizierung wurde die Schwärzung der Proteinbanden densitomtetrisch bestimmt (E. Reihaus, M. Kohler, S. Kraiss, M. Oren, M. Montenarli, Oncogene 5, 137-144 (1990)).
Abbildung 6 zeigt, daß eine Zugabe von 10 µM Antisense-Oligonucleotid zum Expressionssystem eine ca. 70 %ige Inhibierung bewirkt. Bei Erniedrigung der Konzentration auf µM ist nahezu keine Inhibierung mehr festzustellen, ebenso bei Zugabe der Sense-Sequenz, die nicht an die mRNA bindet.

**Patentansprüche**

**Patentansprüche für folgende Vertragsstaaten : AT, BE, CH, DE, DK, FR, GB, IT, LI, LU, NL, SE**

1. Oligonucleotide der Formeln I oder II

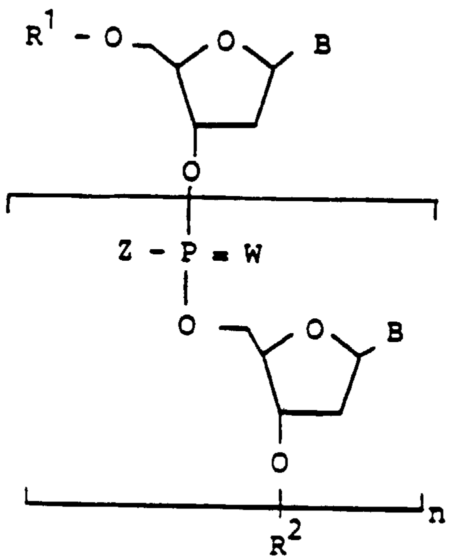

(I)

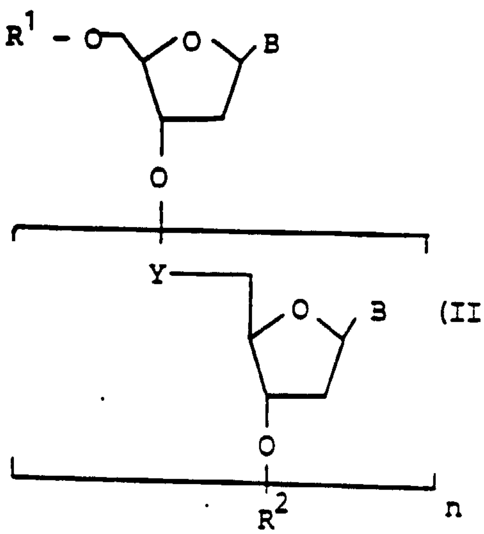

(II)

worin

$R^1$ Wasserstoff oder einen Rest der Formel III bedeutet

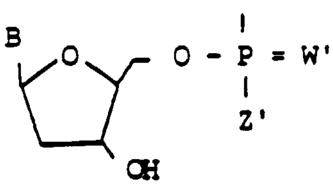

(III)

$R^2$ Wasserstoff oder einen Rest der Formel IV bedeutet,

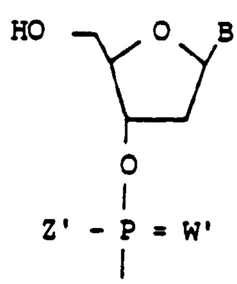

(IV)

wobei jedoch mindestens einer der Reste $R^1$ oder $R^2$ für einen Rest der Formel III oder IV steht;

B für eine Base, wie beispielsweise natürliche Basen wie Adenin, Thymin, Cytosin, Guanin oder unnatürliche Basen, wie beispielsweise Purin, 2,6-Diaminopurin, 7-Deazaadenin, 7-Deazaguanin, $N^4$, $N^4$-Ethanocytosin bzw. deren Prodrugformen steht;

W und W' unabhängig voneinander Sauerstoff oder Schwefel bedeuten;

Z und Z' unabhängig voneinander $O^-$; $S^-$; $C_1$-$C_{18}$-Alkoxy, bevorzugt $C_1$-$C_8$-Alkoxy, besonders bevorzugt $C_1$-$C_3$-Alkoxy, insbesondere Methoxy; $C_1$-$C_{18}$-Alkyl, bevorzugt $C_1$-$C_8$-Alkyl, besonders bevorzugt $C_1$-$C_3$-Alkyl, insbesondere Methyl; $NHR^3$, mit $R^3$ = vorzugsweise $C_1$-$C_{18}$-Alkyl, besonders bevorzugt $C_1$-$C_8$-Alkyl, insbesondere $C_1$-$C_4$-Alkyl, oder $C_1$-$C_4$-Alkoxy-$C_1$-$C_6$-alkyl, bevorzugt Methoxyethyl; $NR^3R^4$, worin $R^3$ wie oben definiert ist und $R^4$ bevorzugt $C_1$-$C_{18}$-Alkyl, besonders bevorzugt $C_1$-$C_8$-Alkyl, insbesondere $C_1$-$C_4$-Alkyl bedeutet oder worin $R^3$ und $R^4$ zusammen mit dem sie tragenden Stickstoffatom einen 5-6-gliedrigen heterozyklischen Ring bedeutet, der zusätzlich ein weiteres Heteroatom aus der Reihe O, S, N enthalten kann, wie z.B. Morpholin;

Y einen Rest aus der Reihe O-Si(R)$_2$, $OCH_2$, C(O)NR oder O-$CH_2$-C(O) bedeutet, worin R für $C_1$-$C_6$-Alkyl, Aryl oder $C_5$ bzw. $C_6$ Cycloalkyl steht; und

n eine ganze Zahl von 5-60, bevorzugt 10-40 und insbesondere bevorzugt 15-25 bedeutet,

sowie deren physiologisch verträglichen Salze.

**2.** Oligonucleotide der Formel 1 gemaß Anspruch 1, dadurch gekennzeichnet, daß $R^2$ für einen Rest der Formel IV steht und $R^1$ Wasserstoff bedeutet; $R^1$ bzw. $R^2$ für einen Rest der Formeln III bzw. IV steht; oder $R^2$ Wasserstoff bedeutet und $R^1$ für einen Rest der Formel III steht, wobei entweder W oder Z im letzten Fall nicht Sauerstoff bedeuten.

**3.** Oligonucleotide der Formel I, gemäß den Ansprüchen 1 oder 2, dadurch gekennzeichnet, daß W für Sauerstoff steht, oder Z und W beide für Sauerstoff stehen.

**4.** Oligonucleotide der Formel I, gemäß den Ansprüchen 1 bis 3, dadurch gekennzeichnet, daß $R^2$ für einen Rest der Formel IV steht und $R^1$ Wasserstoff bedeutet.

**5.** Oligonucleotide der Formeln I und II, gemäß den Ansprüchen 1 bis 4, dadurch gekennzeichnet, daß diese zusätzlich durch Gruppen substituiert sind, die die intrazelluläre Aufnahme begünstigen, die in vitro oder in vivo als Reportergruppen dienen, und/oder Gruppen, die bei der Hybridisierung des Oligonucleotids an biologische DNA oder RNA diese DNA- oder RNA Moleküle unter Bindung oder Spaltung angreifen.

**6.** Verfahren zur Herstellung der Oligonucleotide der Formel I, gemäß den Ansprüchen 1 bis 5, dadurch gekennzeichnet, daß man

a) eine Nucleotideinheit mit 3'- bzw. 5'-terminaler Phosphor(III)- oder Phosphor(V)-Gruppierungen oder deren aktiviertes Derivat mit einer weiteren Nucleotideinheit mit 3'- bzw. 5'-terminaler freier Hydroxygruppe umsetzt oder

b) das Oligonucleotid durch Fragmente in gleicher Weise aufbaut, in den nach (a) oder (b) erhaltenen Oligonucleotiden gegebenenalls eine oder mehrere zum Schutz anderer Funktionen temporär eingeführte Schutzgruppen abspaltet und die so erhaltenen Oligonucleolide der Formel I gegebenenfalls in ihr physiologisch verträgliches Salz überführt.

**7.** Oligonucleotide der Formeln I und II, gemäß den Ansprüchen 1 bis 5 zur Anwendung für hybridisierungschemische, auf der Anlagerung an doppel- oder einzelsträngige Nucleinsäuren beruhende Verfahren der Regulation oder Unterdrückung der biologischen Funktion von Nucleinsäuren sowie zur selektiven Unterdrückung der Expression viraler Genomfunktionen und zur Prophylaxe und Therapie von Virusfunktrionen, zur Unterdrückung der Onkogenfunktion und zur Therapie von Krebserkrankungen.

**8.** Arzneimittel enthaltend eine oder mehrere der Verbindungen gemäß den Ansprüchen 1-5, gegebenenfalls zusammen mit physiologisch verträglichen Hilfs- und/oder Trägerstoffen vorzugsweise für die intravenöse oder topische Verabreichung.

**Patentansprüche für folgende Vertragsstaaten : ES, GR**

1. Verfahren zur Herstellung der Oligonucleotide der Formeln I oder II

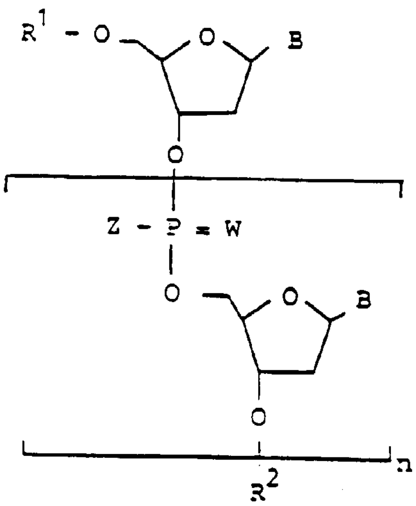

(I)

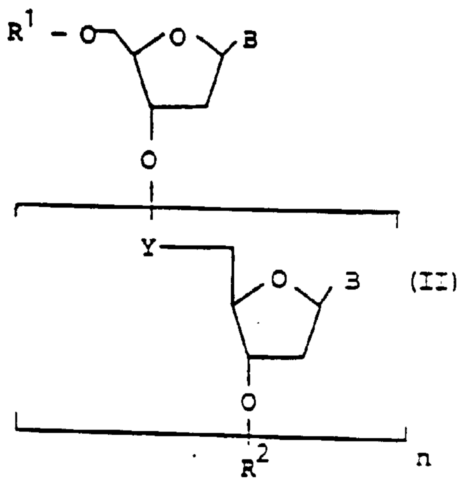

(II)

worin

$R^1$ Wasserstoff oder einen Rest der Formel III bedeutet

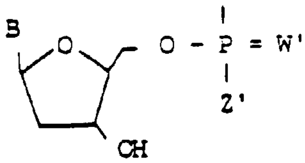

(III)

$R^2$ Wasserstoff oder einen Rest der Formel IV bedeutet,

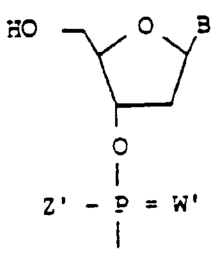

(IV)

wobei jedoch mindestens einer der Reste $R^1$ oder $R^2$ für einen Rest der Formel III oder IV steht;

B für eine Base, wie beispielsweise natürliche Basen wie Adenin, Thymin, Cytosin, Guanin oder unnatürliche Basen, wie beispielsweise Purin, 2,6-Diaminopurin, 7-Deazaadenin, 7-Deazaguanin, $N^4$,$N^4$-Ethanocytosin bzw. deren Prodrugformen steht;

W und W' unabhängig voneinander Sauerstoff oder Schwefel bedeuten;

Z und Z' unabhängig voneinander $O^-$; $S^-$; $C_1$-$C_{18}$-Alkoxy, bevorzugt $C_1$-$C_8$-Alkoxy, besonders bevor-

zugt $C_1$-$C_3$-Alkoxy, insbesondere Methoxy; $C_1$-$C_{18}$-Alkyl, bevorzugt $C_1$-$C_8$-Alkyl, besonders bevorzugt $C_1$-$C_3$-Alkyl, insbesondere Methyl; $NHR^3$, mit $R^3$ = vorzugsweise $C_1$-$C_{18}$-Alkyl besonders bevorzugt $C_1$-$C_8$-Alkyl, insbesondere $C_1$-$C_4$-Alkyl, oder $C_1$-$C_4$-Alkoxy-$C_1$-$C_6$alkyl, bevorzugt Methoxyethyl; $NR^3R^4$, worin $R^3$ wie oben definiert ist und $R^4$ bevorzugt $C_1$-$C_{18}$-Alkyl, besonders bevorzugt $C_1$-$C_8$-Alkyl, insbesondere $C_1$-$C_4$-Alkyl bedeutet oder worin $R^3$ und $R^4$ zusammen mit dem sie tragenden Stickstoffatom einen 5-6-gliedrigen heterozyklischen Ring bedeutet, der zusätzlich ein weiteres Heteroatom aus der Reihe O, S, N enthalten kann, wie z.B. Morpholin;

Y einen Rest aus der Reihe O-Si(R)$_2$, $OCH_2$, C(O)NR oder O-$CH_2$-C(O) bedeutet, worin R für $C_1$-$C_6$-Alkyl, Aryl oder $C_5$ bzw. $C_6$ Cycloalkyl steht; und

n eine ganze Zahl von 5-60, bevorzugt 10-40 und insbesondere bevorzugt 15-25 bedeutet,

sowie deren physiologisch verträglichen Salze, dadurch gekennzeichnet, daß diese zusätzlich durch Gruppen substituiert sind, die die intrazelluläre Aufnahme begünstigen, die in vitro oder in vivo als Reportergruppen dienen, und/oder Gruppen, die bei der Hybridisierung des Oligonucleotids an biologische DNA oder RNA diese DNA- oder RNA Moleküle unter Bindung oder Spaltung angreifen.

2. Verfahren zur Herstellung der Oligonucleotide der Formel I gemäß Anspruch 1, worin $R^2$ für einen Rest der Formel IV steht und $R^1$ Wasserstoff bedeutet; $R^1$ bzw. $R^2$ für einen Rest der Formeln III bzw. IV steht; oder $R^2$ Wasserstoff bedeutet und $R^1$ für einen Rest der Formel III steht, wobei entweder W oder Z im letzten Fall nicht Sauerstoff bedeuten.

3. Verfahren zur Herstellung der Oligonucleotide der Formel I, gemäß den Ansprüchen 1 oder 2, dadurch gekennzeichnet, daß W für Sauerstoff steht, oder Z und W beide für Sauerstoff stehen.

4. Verfahren zur Herstellung der Oligonucleotide der Formel I, gemäß den Ansprüchen 1 bis 3, dadurch gekennzeichnet, daß $R^2$ für einen Rest der Formel IV steht und $R^1$ Wasserstoff bedeutet.

5. Verfahren zur Herstellung der Oligonucleotide der Formeln I und II, gemäß den Ansprüchen 1 bis 4, dadurch gekennzeichnet, daß diese zusätzlich durch Gruppen substituiert sind, die die intrazelluläre Aufnahme begünstigen, die in vitro oder in vivo als Reportergruppen dienen, und/oder Gruppen, die bei der Hybridisierung des Oligonucleotids an biologische DNA oder RNA diese DNA- oder RNA Moleküle unter Bindung oder Spaltung angreifen.

6. Verfahren zur Herstellung einer Zubereitung enthaltend eine oder mehrere der Verbindungen gemäß den Ansprüchen 1-5, gegebenenfalls zusammen mit physiologisch verträglichen Hilfs- und/oder Trägerstoffen vorzugsweise für die intravenöse oder topische Verabreichung, dadurch gekennzeichnet, daß man diese und einen oder mehrere Träger in eine geeignete Darreichungsform bringt.

## Claims

**Claims for the following Contracting States : AT, BE, CH, DE, DK, FR, GB, IT, LI, LU, NL, SE**

1. An oligonucleotide of the formula I or II

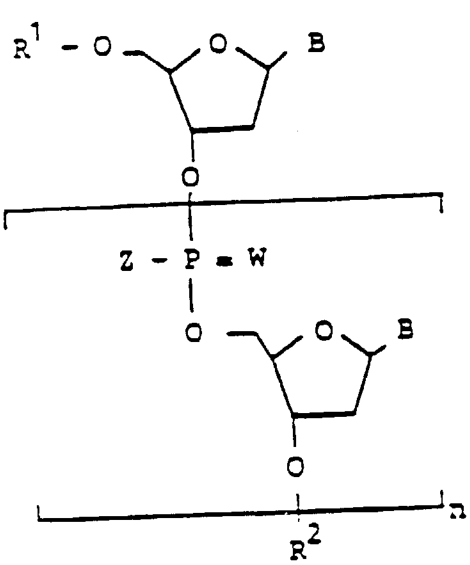

(I)

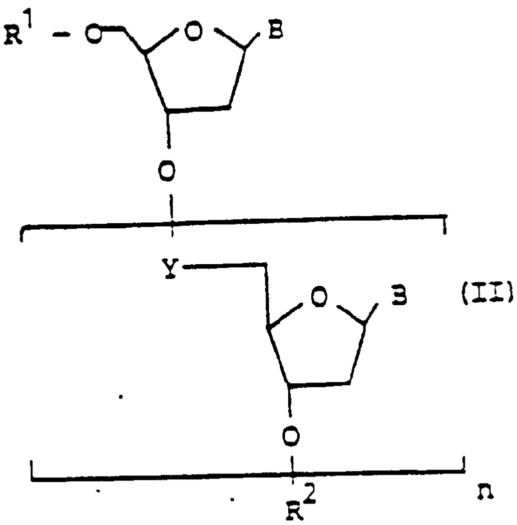

(II)

in which

$R^1$ is hydrogen or a radical of the formula III

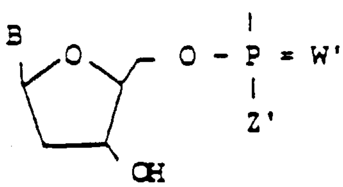

(III)

$R^2$ is hydrogen or a radical of the formula IV

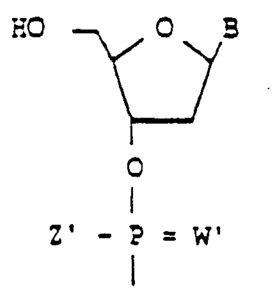

(IV)

but where at least one of the radicals $R^1$ or $R^2$ is a radical of the formula III or IV;

B is a base such as, for example, natural bases such as adenine, thymine, cytosine, guanine or unnatural bases such as, for example, purine, 2,6-diaminopurine, 7-deazaadenine, 7-deazaguanine or $N^4$,$N^4$-ethanocytosine or their prodrug forms;

W and W' are, independently of one another, oxygen or sulfur;

Z and Z' are, independently of one another, $O^-$; $S^-$; $C_1$-$C_{18}$-alkoxy, preferably $C_1$-$C_8$-alkoxyl particularly preferably $C_1$-$C_3$-alkoxy, especially methoxy; $C_1$-$C_{18}$-alkyl, preferably $C_1$-$C_8$-alkyl, particularly preferably $C_1$-$C_3$-alkyl, especially methyl; $NHR^3$ with $R^3$ = preferably $C_1$-$C_{18}$-alkyl, particularly preferably $C_1$-$C_8$-alkyl, especially $C_1$-$C_4$-alkyl, or $C_1$-$C_4$-alkoxy-$C_1$-$C_6$-alkyl, preferably methoxyethyl; $NR^3R^4$ in which $R^3$ is as defined above and $R^4$ is preferably $C_1$-$C_{18}$-alkyl, particularly preferably $C_1$-$C_8$-alkyl, especially $C_1$-$C_4$-alkyl, or in which $R^3$ and $R^4$ is, together with the nitrogen atom carrying them, a 5-6-membered heterocyclic ring which can additionally contain another hetero atom from the series comprising O, S, N,

such as, for example, morpholine;

Y is a radical from the series comprising O-Si(R)$_2$, $OCH_2$, C(O)NR or O-$CH_2$-C(O) in which R is $C_1$-$C_6$-alkyl, aryl or $C_5$- or $C_6$-cycloalkyl; and

n is an integer from 5 to 60, preferably 10-40 and particularly preferably 15-25,

and the physiologically tolerated salts thereof.

**2.** An oligonucleotide of the formula I as claimed in claim 1, wherein $R^2$ is a radical of the formula IV and $R^1$ is hydrogen; $R^1$ and $R^2$ are a radical of the formulae III and IV respectively; or $R^2$ is hydrogen and $R^1$ is a radical of the formula III where either W or Z in the latter case is not oxygen.

**3.** An oligonucleotide of the formula I as claimed in claim 1 or 2, wherein W is oxygen or both Z and W are oxygen.

**4.** An oligonucleotide of the formula I as claimed in claims 1 to 3, wherein $R^2$ is a radical of the formula IV, and $R^1$ is hydrogen.

**5.** An oligonucleotide of the formula I or II as claimed in claims 1 to 4, which is additionally substituted by groups which favor intracellular uptake, which act in vitro or in vivo as reporter groups, and/or groups which on hybridization of the oligonucleotide with biological DNA or RNA attack these DNA or RNA molecules, with bond formation or cleavage.

**6.** A process for preparing the oligonucleotides of the formula I as claimed in claims 1 to 5, which comprises

a) reacting a nucleotide unit with 3'- or 5'-terminal phosphorus (III) or phosphorus (V) groups or the activated derivative thereof with another nucleotide unit with free 3'- or 5'-terminal hydroxyl group or
b) synthesizing the oligonucleotide by fragments in the same way, where appropriate eliminating one or more protective groups temporarily introduced into the oligonucleotides obtained as in (a) or (b) to protect other functions, and, where appropriate, converting the oligonucleotides of the formula I obtained in this way into their physiologically tolerated salt.

**7.** An oligonucleotide of the formula I or II as claimed in claims 1 to 5, for use for chemical hybridization methods, which are based on the attachment to double- or single-stranded nucleic acids, for regulation or suppression of the biological function of nucleic acids, and for selective suppression of the expression of viral genome functions and for the prophylaxis and therapy of viral diseases, for suppressing oncogene function and for therapy of cancers.

**8.** A pharmaceutical containing one or more of the compounds as claimed in claims 1-5, where appropriate together with physiologically tolerated auxiliaries and/or vehicles, preferably for intravenous or topical administration.

**Claims for the following Contracting States : ES, GR**

**1.** A process for preparing oligonucleotides of the formulae I or II

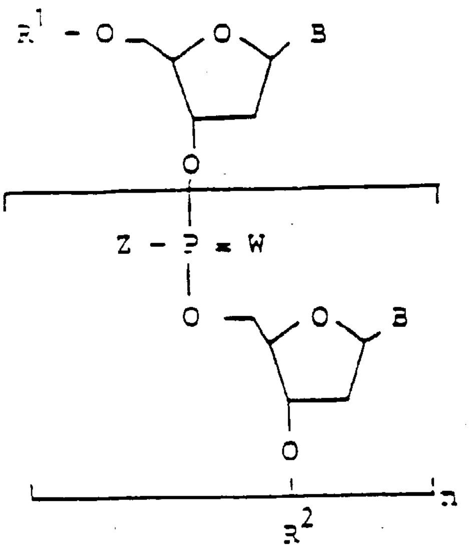

(I)

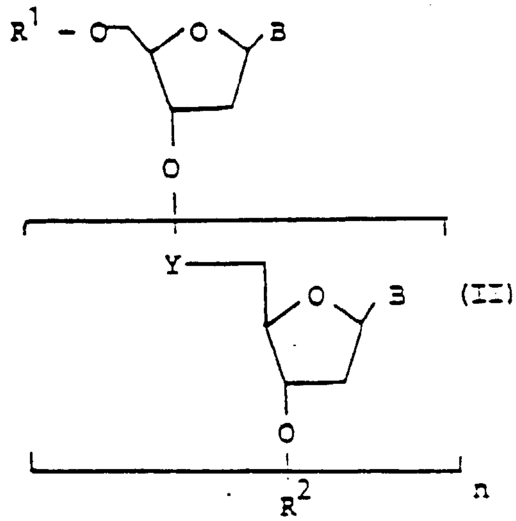

(II)

in which

$R^1$ is hydrogen or a radical of the formula III

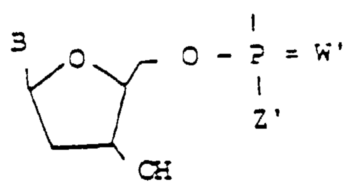

(III)

$R^2$ is hydrogen or a radical of the formula IV

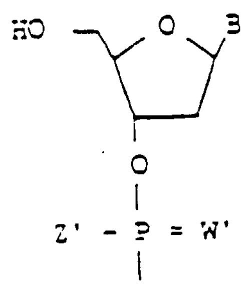

(IV)

but where at least one of the radicals $R^1$ or $R^2$ is a radical of the formula III or IV;

B is a base such as, for example, natural bases such as adenine, thymine, cytosine, guanine or unnatural bases such as, for example, purine, 2,6-diaminopurine, 7-deazaadenine, 7-deazaguanine or $N^4$,$N^4$-ethanocytosine or their prodrug forms;

W and W' are, independently of one another, oxygen or sulfur;

Z and Z' are, independently of one another, $O^-$; $S^-$; $C_1$-$C_{18}$-alkoxy, preferably $C_1$-$C_8$-alkoxy, particularly preferably $C_1$-$C_3$-alkoxy, especially methoxy; $C_1$-$C_{18}$-alkyl, preferably $C_1$-$C_8$-alkyl, particularly preferably $C_1$-$C_3$-alkyl, especially methyl; $NHR^3$ with $R^3$ = preferably $C_1$-$C_{18}$-alkyl, particularly preferably $C_1$-$C_8$-alkyl, especially $C_1$-$C_4$-alkyl, or $C_1$-$C_4$-alkoxy-$C_1$-$C_6$-alkyl, preferably methoxyethyl; $NR^3R^4$ in which $R^3$ is as defined above and $R^4$ is preferably $C_1$-$C_{18}$-alkyl, particularly preferably $C_1$-$C_8$-alkyl, especially $C_1$-$C_4$-alkyl, or in which $R^3$ and $R^4$ is, together with the nitrogen atom carrying them, a 5-6-membered heterocyclic ring which can additionally contain another hetero atom from the series comprising O, S, N, such as, for example, morpholine;

y is a radical from the series comprising $O-Si(R)_2$, $OCH_2$, $C(O)NR$ or $O-CH_2-C(O)$ in which R is $C_1$-$C_6$-alkyl, aryl or $C_5$- or $C_6$-cycloalkyl; and

n is an integer from 5 to 60, preferably 10-40 and particularly preferably 15-25,

and the physiologically tolerated salts thereof wherein these are additionally substituted by groups which favor intracellular uptake, which act in vitro or in vivo as reporter groups, and/or groups which on hybridization of the oligonucleotide with biological DNA or RNA attack this DNA or RNA molecule, with bond formation or cleavage.

**2.** A process for preparing oligonucleotides of the formula I as claimed in claim 1, in which $R^2$ is a radical of the formula IV and $R^1$ is hydrogen; $R^1$ and $R^2$ are a radical of the formulae III and IV respectively; or $R^2$ is hydrogen and $R^1$ is a radical of the formula III where either W or Z in the latter case is not oxygen.

**3.** A process for preparing oligonucleotides of the formula I as claimed in claim 1 or 2, wherein W is oxygen or both Z and W are oxygen.

**4.** A process for preparing oligonucleotides of the formula I as claimed in claims 1 to 3, wherein $R^2$ is a radical of the formula IV, and $R^1$ is hydrogen.

**5.** A process for preparing oligonucleotides of the formulae I and II as claimed in claims 1 to 4, wherein these are additionally substituted by groups which favor intracellular uptake, which act in vitro or in vivo as reporter groups, and/or groups which on hybridization of the oligonucleotide with biological DNA or RNA attack these DNA or RNA molecules, with bond formation or cleavage.

**6.** A process for producing a preparation containing one or more of the compounds as claimed in claims 1-5, where appropriate together with physiologically tolerated auxiliaries and/or vehicles, preferably for intravenous or topical administration, which comprises bringing these and one or more carriers into a suitable dosage from.

**Revendications**

**Revendications pour les Etats contractants suivants : AT, BE, CH, DE, DK, FR, GB, IT, LI, LU, NL, SE**

**1.** Oligonucléotides de formule I ou II

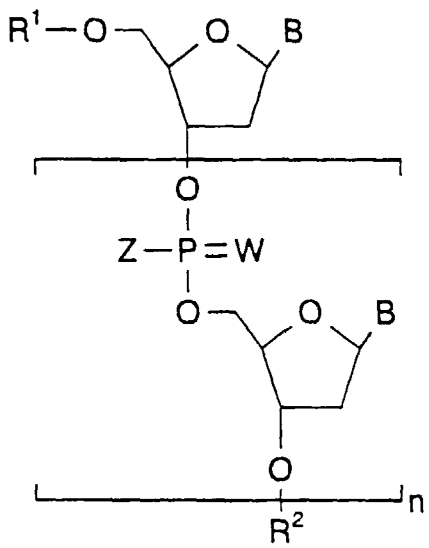

(I)

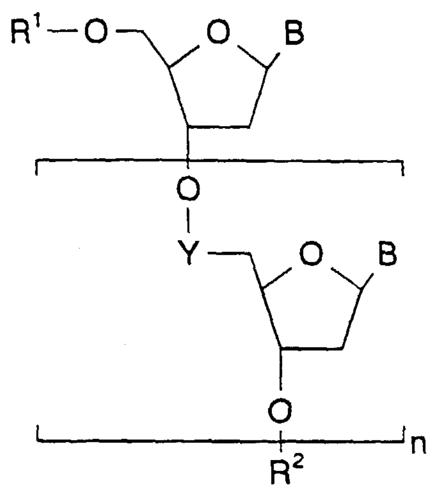

(II)

dans laquelle

$R^1$ signifie un atome d'hydrogène ou un reste de formule III

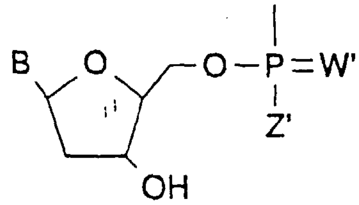

(III)

$R^2$ signifie un atome d'hydrogène ou un reste de formule IV

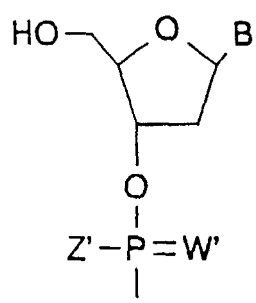

(IV)

où au moins un des groupes $R^1$ ou $R^2$ représente un reste de formule III ou IV ;

B représente une base, comme par exemple des bases naturelles telles que l'adénine, la thymine, la cytosine, la guanine, ou des bases synthétiques, telles que la purine, la 2,6-diaminopurine, la 7-déaza-adénine, la 7-déazaguanine, la $N^4$,$N^4$-éthanocytosine ou leurs formes précurseur ;

W et W', indépendants l'un de l'autre, signifient un atome d'oxygène ou de soufre ;

Z et Z', indépendants l'un de l'autre, signifient $O^-$, $S^-$, un groupe alcoxy $C_1$-$C_{18}$, de préférence alcoxy $C_1$-$C_8$, en particulier alcoxy $C_1$-$C_3$, tout particulièrement méthoxy; un groupe alkyle $C_1$-$C_{18}$, de préférence alkyle $C_1$-$C_8$, en particulier alkyle $C_1$-$C_3$, tout particulièrement méthyle ; $NHR^3$, avec $R^3$ = de préférence un groupe alkyle $C_1$-$C_{18}$, en particulier alkyle $C_1$-$C_8$, tout particulièrement alkyle $C_1$-$C_4$, ou un groupe alcoxy-$C_1$-$C_4$-alkyle $C_1$-$C_6$, de préférence méthoxyéthyle; $NR^3R^4$ où $R^3$ est défini comme précédemment et $R^4$ est de préférence un groupe alkyle $C_1$-$C_{18}$, en particulier alkyle $C_1$-$C_8$, tout particulièrement alkyle $C_1$-$C_4$ et où $R^3$ et $R^4$ peuvent signifier ensemble, avec l'atome d'azote qu'ils portent, un hétérocycle à 5 ou 6 atomes qui peut contenir un hétéroatome supplémentaire parmi O, S, N, par exemple le groupe morpholine ;

Y signifie un reste parmi $O\text{-}Si(R)_2$, $OCH_2$, C(O)NR ou $O\text{-}CH_2\text{-}C(O)$, où R représente un groupe alkyle $C_1$-$C_6$, aryle ou cycloalkyle $C_5$ ou $C_6$ ; et

n signifie un nombre entier de 5 à 60, de préférence de 10 à 40, en particulier de 15 à 25,

ainsi que leurs sels physiologiquement acceptables.

**2.** Oligonudéotidés de formule I selon la revendication 1, caractérisés en ce que $R^2$ représente un reste de formule IV et $R^1$ signifie un atome d'hydrogène ; $R^1$ et $R^2$ respectivement représentent un reste de formule III et IV respectivement ; ou $R^2$ signifie un atome d'hydrogène et $R^1$ représente un reste de formule III, dans ce dernier cas, W ou Z ne signifient pas un atome d'oxygène.

**3.** Oligonucléotides de formule I selon la revendication 1 ou 2, caractérisés en ce que W représente un atome d'oxygène ou Z et W représentent tous les deux un atome d'oxygène.

**4.** Oligonucléotides de formule I selon les revendications 1 à 3, caractérisés en ce que $R^2$ représente un reste de formule IV et $R^1$ signifie un atome d'hydrogène.

**5.** Oligonucléotides de formule I et II selon les revendications 1 à 4, caractérisé en ce qu'ils sont de plus substitués

par des groupes qui favorisent l'absorption intracellulaire, qui servent de groupes marqueurs *in vitro* ou *in vivo* et/ou des groupes qui affectent par liaison ou clivage les molécules d'ADN ou d'ARN lors de l'hybridation de l'oligonucléotide sur de l'ADN ou de l'ARN biologique.

**6.** Procédé pour la préparation des oligonucléotides de formule I selon les revendications 1 à 5, caractérisé en ce que

a) on fait réagir une unité de nucléotide à groupes phosphore(III) ou phosphore(V) 3' ou 5' terminaux ou son dérivé activé avec une autre unité de nucléotide à groupe hydroxy libre à l'extrémité 3' ou 5' ou
b) on construit l'oligonudéotide, de la même façon, par fragments, on élimine éventuellement, dans les oligonucléotides obtenus en a) ou en b), un ou plusieurs groupes protecteurs temporaires introduits pour la protection d'autres fonctions et on transforme éventuellement les oligonucléotides de formule I ainsi obtenus en leurs sels physiologiquement acceptables.

**7.** Oligonucléotides de formule I et II selon les revendications 1 à 5 pour leur utilisation dans des procédés chimiques d'hybridation pour la régulation ou la répression des fonctions biologiques d'acides nucléiques, basés sur la fixation à des acides nucléiques double brin ou à simple brin, ainsi que pour la répression sélective de l'expression de fonctions génomiques virales et pour la prophylaxie et le traitement de fonctions virales, pour la répression des fonctions oncogènes et pour le traitement de maladies cancéreuses.

**8.** Médicament contenant un ou plusieurs composés selon les revendications 1 à 5, éventuellement avec des adjuvants et/ou des véhicules physiologiquement acceptables, de préférence pour l'administration intraveineuse ou topique.

**Revendications pour les Etats contractants suivants : ES, GR**

**1.** Procédé de préparation des oligonucléotides de formule I ou II

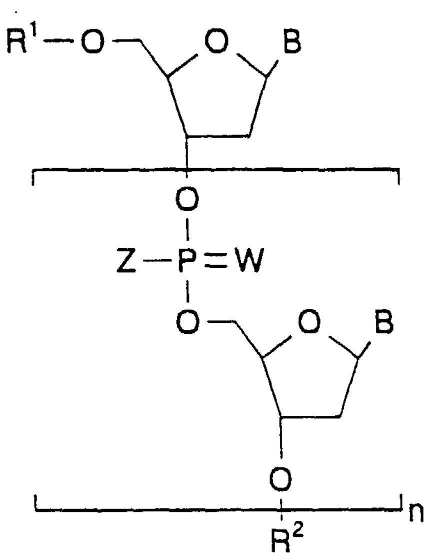
(I)

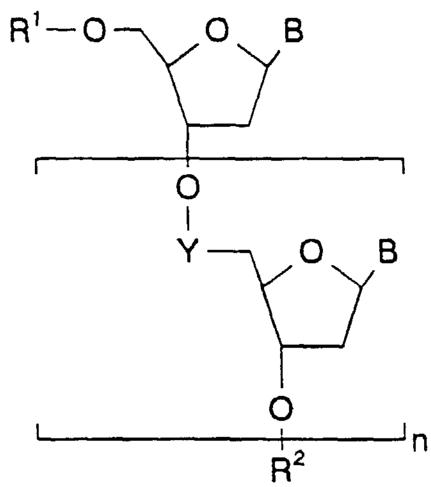
(II)

dans laquelle

$R^1$ signifie un atome d'hydrogène ou un reste de formule III

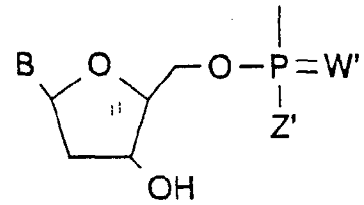

(III)

$R^2$ signifie un atome d'hydrogène ou un reste de formule IV

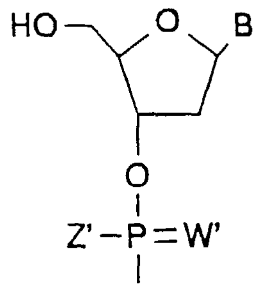

(IV)

où au moins un des groupes $R^1$ ou $R^2$ représente un reste de formule III ou IV ;

B représente une base, comme par exemple des bases naturelles telles que l'adénine, la thymine, la cytosine, la guanine, ou des bases synthétiques, telles que la purine, la 2,6-diaminopurine, la 7-déaza-adénine, la 7-déazaguanine, la $N^4$,$N^4$-éthanocytosine ou leurs formes précurseur ;

W et W', indépendants l'un de l'autre, signifient un atome d'oxygène ou de soufre;

Z et Z', indépendants l'un de l'autre, signifient $O^-$, $S^-$, un groupe alcoxy $C_1$-$C_{18}$, de préférence alcoxy $C_1$-$C_8$, en particulier alcoxy $C_1$-$C_3$, tout particulièrement méthoxy ; un groupe alkyle $C_1$-$C_{18}$, de préférence alkyle $C_1$-$C_8$, en particulier alkyle $C_1$-$C_3$, tout particulièrement méthyle ; $NHR^3$, avec $R^3$ = de préférence un groupe alkyle $C_1$-$C_{18}$, en particulier alkyle $C_1$-$C_8$, tout particulièrement alkyle $C_1$-$C_4$, ou un groupe alcoxy-$C_1$-$C_4$-alkyle $C_1$-$C_6$, de préférence méthoxyéthyle ; $NR^3R^4$ où $R^3$ est défini comme précédemment et $R^4$ est de préférence un groupe alkyle $C_1$-$C_{18}$, en particulier alkyle $C_1$-$C_8$, tout particulièrement alkyle $C_1$-$C_4$ et où $R^3$ et $R^4$ peuvent signifier ensemble, avec l'atome d'azote qu'ils portent, un hétérocycle à 5 ou 6 atomes qui peut contenir un hétéroatome supplémentaire parmi O, S, N, par exemple le groupe morpholine ;

Y signifie un reste parmi O-Si(R)$_2$, $OCH_2$, C(O)NR ou O-$CH_2$-C(O), où R représente un groupe alkyle $C_1$-$C_6$, aryle ou cycloalkyle $C_5$ ou $C_6$ ; et

n signifie un nombre entier de 5 à 60, de préférence de 10 à 40, en particulier de 15 à 25,

ainsi que leurs sels physiologiquement acceptables, caractérisé en ce qu'ils sont de plus substitués par des groupes qui favorisent l'absorption intracellulaire, qui servent de groupes marqueurs *in vitro* ou *in vivo* et/ou des groupes qui affectent par liaison ou clivage les molécules d'ADN ou d'ARN lors de l'hybridation de l'oligonudéotide sur de l'ADN ou de l'ARN biologique.

**2.** Procédé pour la préparation des oligonucléotides de formule I selon la revendication 1, dans laquelle $R^2$ représente un reste de formule IV et $R^1$ signifie un atome d'hydrogène ; $R^1$ et $R^2$ respectivement représentent un reste de formule III et IV respectivement ; ou $R^2$ signifie un atome d'hydrogène et $R^1$ représente un reste de formule III, dans ce dernier cas, W ou Z ne signifient pas un atome d'oxygène.

**3.** Procédé pour la préparation des oligonucléotides de formule I selon la revendication 1 ou 2, caractérisé en ce que W représente un atome d'oxygène ou Z et W représentent tous les deux un atome d'oxygène.

**4.** Procédé pour la préparation des oligonucléotides de formule I selon les revendications 1 à 3, caractérisé en ce que $R^2$ représente un reste de formule IV et $R^1$ signifie un atome d'hydrogène.

**5.** Procédé pour la préparation des oligonucléotides de formule I et II selon les revendications 1 à 4, caractérisé en

ce que ceux-ci sont de plus substitués par des groupes qui favorisent l'absorption intracellulaire, qui servent de groupes marqueurs *in vitro* ou *in vivo* et/ou des groupes qui affectent par liaison ou clivage les molécules d'ADN ou d'ARN lors de l'hybridation de l'oligonudéotide sur de l'ADN ou de l'ARN biologique.

**6.** Procédé pour la fabrication d'une préparation contenant un ou plusieurs composés selon les revendications 1 à 5, éventuellement avec des adjuvants et/ou des véhicules physiologiquement acceptables, de préférence pour l'administration intraveineuse ou topique, caractérisé en ce que l'on met ceux-ci, avec un ou plusieurs véhicules, sous une forme d'administration appropriée.

## Fig. 1:

AUTORADIOGRAMM DER SEQUENZIERUNG VON SV40TS17, SV40TAS17 UND SV40TAS17 (3'-3', 5'-5')

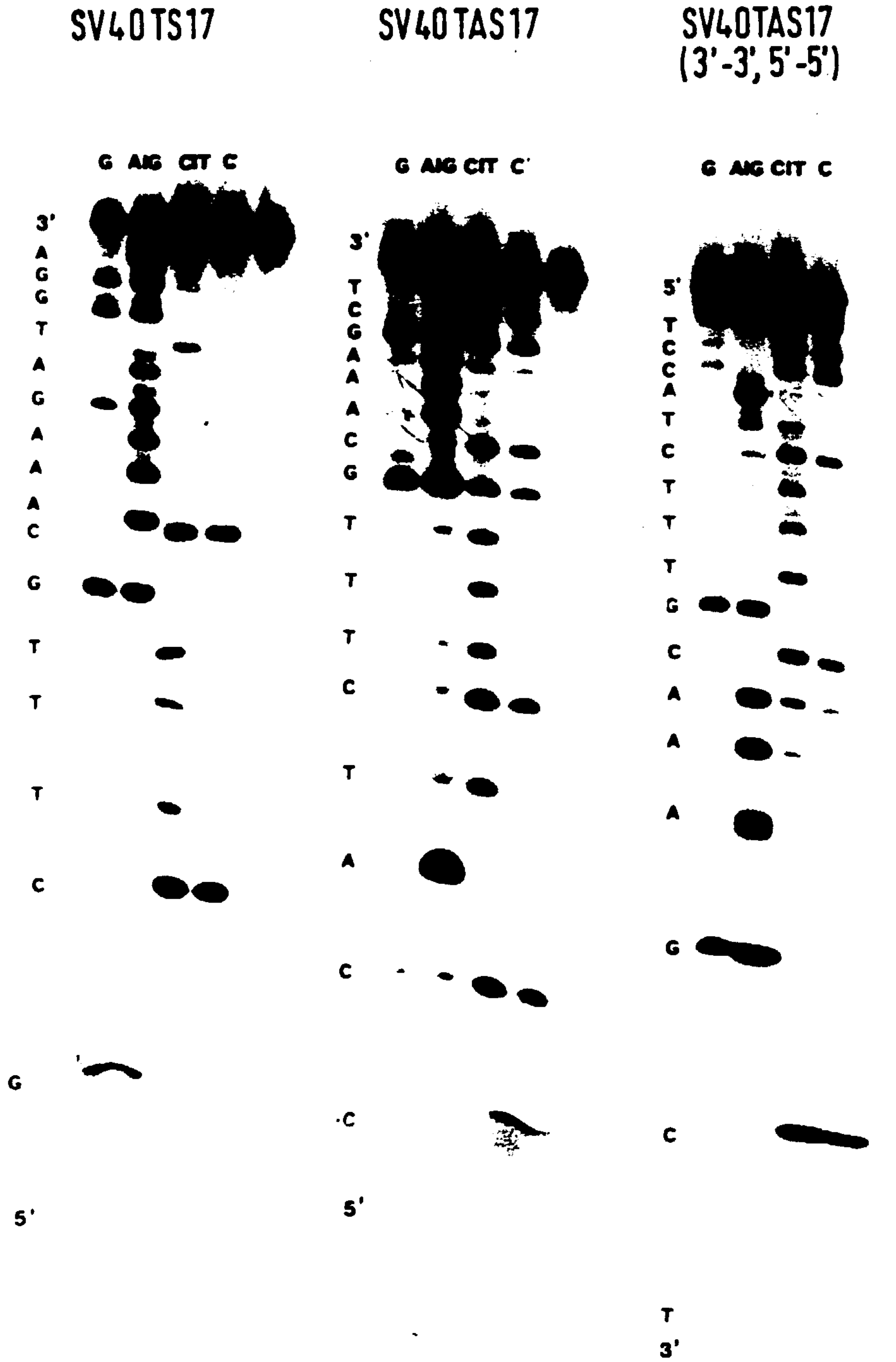

**Fig. 2:**

AUTORADIOGRAMM DER SPALTUNG VON $dT_{20}$ BZW.
$dT_{20}$ (3'-3', 5'-5') MIT HUMANSERUM
VON LINKS:
$dT_{20}$ : REAKTION NACH 0, 5, 8, 11, 15 UND 30 MINUTEN
$dT_{20}$ (3'-3', 5'-5'): REAKTION NACH 0, 5, 8, 11, 25, 30 UND 90 MINUTEN

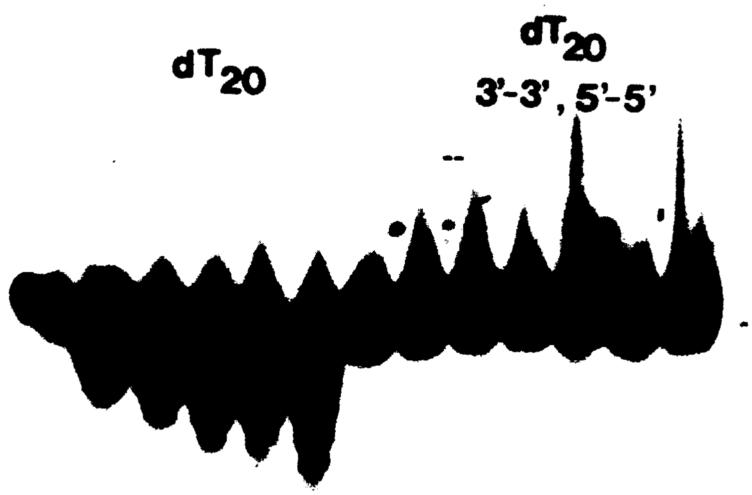

Fig. 3:

AUTORADIOGRAMM DER SVpdE-SPALTUNG: VON LINKS: $dT_{20}$, $dT_{20}$(3'-3', 5'-5'), SPALTUNG VON $dT_{20}$(3'-3', 5'-5') NACH 5, 15, 30, 45, 60 UND 90 MIN., MIX AUS 0 MIN./15 MIN., MIX AUS 0 MIN./30 MIN., SPALTUNG VON $dT_{20}$ NACH 5, 15, 30 UND 45 MINUTEN.

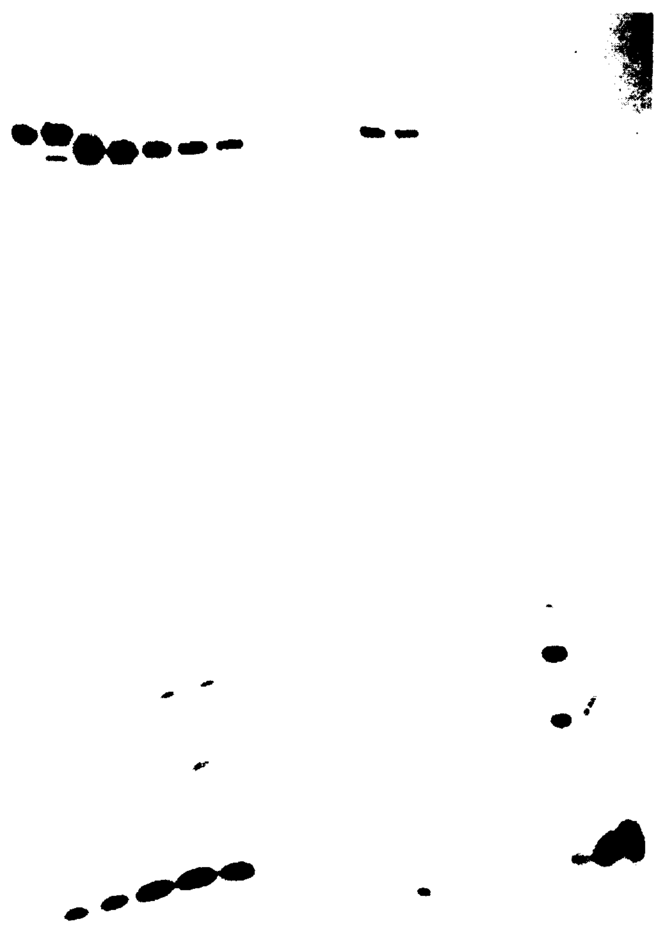

Fig. 4:

MODULATION DER EXPRESSION VON T-Ag DURCH ANTISENSE - OLIGONUCLEOTIDE.
LINIE 1: ZELLYSAT OHNE INHIBIERUNG MIT 30 μM SV4DTAS17 (3'-3', 5'-5')
LINIE 2: ZELLYSAT OHNE INHIBIERUNG
M : LÄNGENMARKER

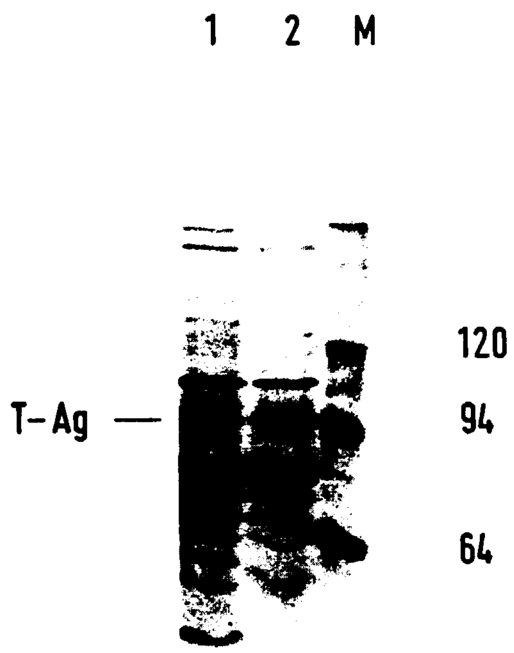

Fig. 5

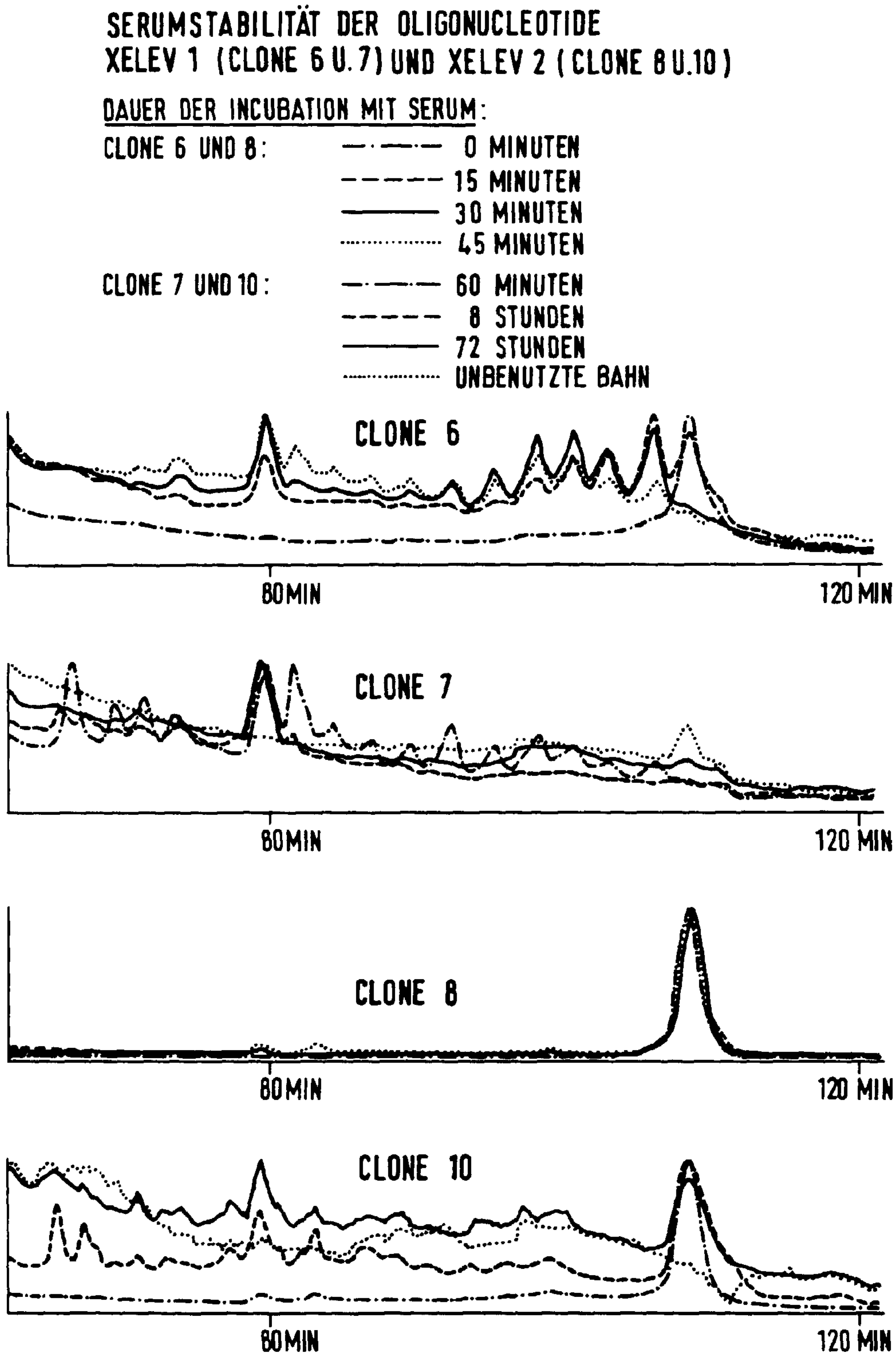
SERUMSTABILITÄT DER OLIGONUCLEOTIDE
XELEV 1 (CLONE 6 U. 7) UND XELEV 2 (CLONE 8 U.10)
DAUER DER INCUBATION MIT SERUM:
CLONE 6 UND 8:
0 MINUTEN
15 MINUTEN
30 MINUTEN
45 MINUTEN
CLONE 7 UND 10:
60 MINUTEN
8 STUNDEN
72 STUNDEN
UNBENUTZTE BAHN
CLONE 6
80MIN
120 MIN
CLONE 7
80MIN
120 MIN
CLONE 8
80MIN
120 MIN
CLONE 10
80MIN
120 MIN

HEMMUNG DER BIOSYNTHESE DES ONCOPROTEINS p 53
DURCH EIN ANTISENSE - INV-OLIGONUCLEOTID

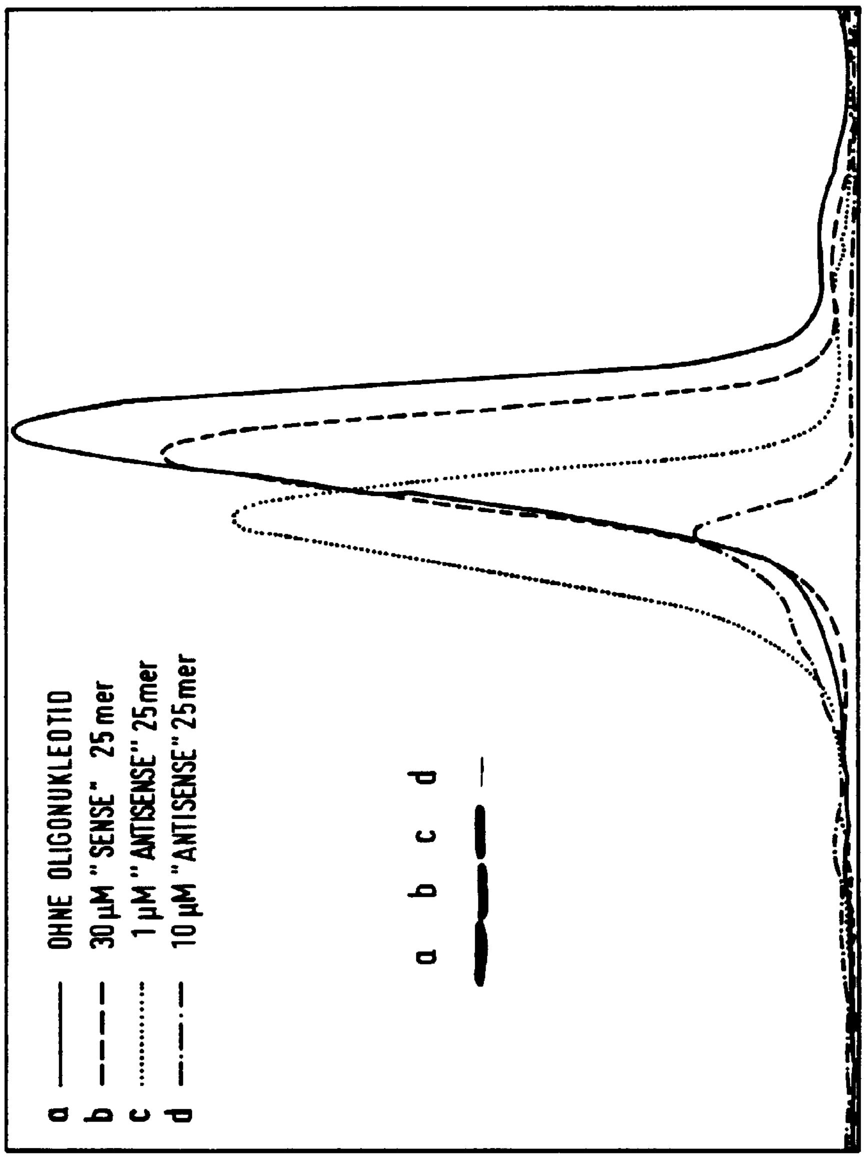


Fig. 6